# NEUE EUROPÄISCHE PATENTSCHRIFT

(11) **EP 2 125 812 B2**
(45) Veröffentlichungstag und Bekanntmachung des Hinweises auf die Entscheidung über den Einspruch: **25.05.2016**
(45) Hinweis auf die Patenterteilung: 31.10.2012
(21) Anmeldenummer: 08734313.3
(22) Anmeldetag: 26.02.2008
(51) Int. Cl.: C07D 471/04, A61K 31/437, A61P 35/00

(54) **VERWENDUNG VON ß-CARBOLINEN ZUR BEHANDLUNG VON NEURODEGENERATIVEN ERKRANKUNGEN**
USE OF ß-CARBOLINES FOR THE TREATMENT OF NEURODEGENERATIVE DISEASES
UTILISATION DE ß-CARBOLINES POUR LE TRAITEMENT DE MALADIES NEURODÉGÉNÉRATIVES

(30) Priorität: 26.02.2007 DE 102007009264
(43) Veröffentlichungstag der Anmeldung: 02.12.2009
(73) Patentinhaber: Ellneuroxx Ltd., 41460 Neuss (DE)
(72) Erfinder: ROMMELSPACHER, Hans, 14050 Berlin (DE)
(74) Vertreter: Arth, Hans-Lothar
(86) Internationale Anmeldenummer: PCT/DE2008/000332
(87) Internationale Veröffentlichungsnummer: WO 2008/104161

(56) Entgegenhaltungen:
- EP-A- 0 667 347
- EP-A- 1 634 881
- DE-A1- 3 240 514
- DE-A1- 4 223 164
- DE-B- 1 044 821
- GB-A- 2 155 462
- US-A- 2 767 179
- US-A- 2 850 501
- US-A- 3 663 559
- CAO ET AL: "Design, synthesis and in vitro and in vivo antitumor activities of novel beta-carboline derivatives" EUROPEAN JOURNAL OF MEDICINAL CHEMISTRY, EDITIONS SCIENTIFIQUE ELSEVIER, PARIS, FR, Bd. 40, Nr. 10, 1. Oktober 2005 (2005-10-01), Seiten 991-1001, XP005097816 ISSN: 0223-5234
- BRACHER F ET AL: "Beta-Carbolin-Alkaloide" PHARMAZIE, DIE, GOVI VERLAG, ESCHBORN, DE, Bd. 50, Nr. 3, 1. März 1995 (1995-03-01), Seite 182/183, XP002133529 ISSN: 0031-7144
- BALON M ET AL: "HYDROGEN-BONDING AND PROTON TRANSFER INTERACTIONS BETWEEN HARMANE AND TRIFLUOROETHANOL IN THE GROUND AND EXCITED SINGLET STATES" PHOTOCHEMISTRY AND PHOTOBIOLOGY, OXFORD, GB, Bd. 67, Nr. 4, 1. April 1998 (1998-04-01), Seiten 414-419, XP009036503 ISSN: 0031-8655
- MATSUBARA, KAZUO ET AL: "Endogenously occurring .beta.-carboline induces Parkinsonism in nonprimate animals: a possible causative protoxin in idiopathic Parkinson 's disease" JOURNAL OF NEUROCHEMISTRY ( 1998 ), 70(2), 727-735 CODEN: JONRA9; ISSN: 0022-3042, 1998, XP002489055
- WON TAE CHOI ET AL: "Protective Effect of 1-Methylated [beta]-Carbolines Against 3-Morpholinosydnonimine-Induced Mitochondrial Damage and Cell Viability Loss in PC12 Cells" NEUROCHEMICAL RESEARCH, KLUWER ACADEMIC PUBLISHERS-PLENUM PUBLISHERS, NE, Bd. 29, Nr. 10, 1. Oktober 2004 (2004-10-01), Seiten 1807-1816, XP019289675 ISSN: 1573-6903

## Beschreibung

Die Erfindung betrifft Derivate von β-Carbolinen bevorzugt 9-Alkyl-β-Carboline (9-Alkyl-BC) geeignet zur Verwendung zur Prophylaxe und Behandlung von Bewegungsstörungen sowie Zellkulturmedien, Nährmedien oder Fermentationsmedien enthaltend mindestens eine β-Carbolin-Verbindung als Additivum.

### Hintergrund der Erfindung

EP 1 634 881 A1 offenbart β-Carbolin-Derivate und untersuchte deren zelltoxische Wirkung und Aktivität gegen Tumoren. Cao et al. (Eur J Med Chem. 2005;40:991-1001) stellen β-Carboline vor, die in den Positionen 1, 3 und 9 substituiert sein können. Einige der von Cao et al. offenbarten β-Carbolin-Derivate wurden auf zelltoxische Wirksamkeit und Aktivität gegen Tumoren untersucht. EP 0667347 A1 betrifft β-Carbolin-Derivate, die als Thromboxan-Synthase-Inhibitoren wirken und deshalb als Antikoagulantien zur Vorbeugung und Behandlung verschiedener Erkrankungen im Zusammenhang mit Gefäßverschluss und Entzündungsvorgängen eingesetzt werden. DE 4223164 A1 beschreibt 9-Carbamoyl-β-Carbolin-Derivate sowie das Verfahren zu deren Herstellung und Verwendung in Arzneimitteln. DE 32 40 514 offenbart β-Carboline, die in Position 3 einen Substituenten tragen, der sich nicht von einer Carbonsäure ableitet, oder die in Position 3 unsubstituiert sind. US 2850501 A beschreibt β-Carbolin-Derivate sowie Verfahren zu ihrer Herstellung. Ferner offenbart US 2850501 A, dass die beanspruchten β-Carboline blutdrucksenkende, anti-emetische sowie eine beruhigende Wirkung auf das zentrale Nervensystem ausüben und deshalb zur Behandlung von Zuständen mentaler Erregtheit geeignet sein können. US 3663559 A behandelt die Synthese eines breiten Spektrums von Pyridon-Derivaten. Bracher und Papke (Pharmazie. 1995;50:525-7) beschreiben die Synthese des Alkaloids Infractin, das eine inhibitorische Wirkung auf die cAMP-Phosphodieesterase aufweist. GB 2 155 462 A beschreibt als Herbizide und Fungizide eingesetzte ß-Carboline. Balón et al. (Biospectroscopy. 1998;4:185-95) stellen eine Studie zur Bildung von Wasserstoffbrücken-Bindungen und Protonen-Transferreaktionen von Harman (1-Methyl-β-Carbolin) und seinem 9-Methyl-Derivat vor. US 2767179 A betrifft Harman-(1-Methyl-β-Carbolin)- und Norharman-(β-Carbolin)-Derivaten und deren Synthese, wobei die Derivate in Position 9 eine Alkylenaminoalkylgruppe tragen. DE 1044821 B offenbart die Synthese von 9-Alkyl-β-Carbolinen und speziell von 9-Methyl-β-Carbolin.

Die β-Carbolin Verbindungen sollen insbesondere beim Morbus Parkinson eingesetzt werden. Der Morbus Parkinson, auch als Parkinson-Syndrom oder Parkinson'sche Krankheit bezeichnet, zählt zu den chronischen Krankheiten, welche bisher noch unheilbar sind. Der Krankheitsverlauf zeichnet sich dadurch aus, dass im Gehirn Nervenzellen absterben. Dies betrifft vorwiegend diejenigen Nervenzellen in der substantia nigra, der schwarzen Substanz, die den Botenstoff Dopamin enthalten. Dadurch ist die Bildung des Botenstoffes Dopamin nicht mehr in ausreichendem Maße gewährleistet. Veränderungen (z. B. Lewy-Körperchen, Absterben anderer Nervenzelltypen) sind auch in anderen Hirnteilen nachweisbar, wie z. B. dem nucleus coeruleus, den Raphé-Kernen, dem nucleus basalis Meynert, dem Vaguskem und dem Hippocampus. Dopamin ist ein für die Kontrolle des Bewegungsapparates essentieller Botenstoff, dessen Mangel zu Bewegungsstörungen wie Zittern (Ruhe-Tremor), unwillkürlichen Muskelspannungen (Rigor) und einer Verlangsamung der Bewegung (Hypokinese) führt. In fortgeschrittenen Stadien kommen weitere Bewegungsstörungen hinzu wie die Unfähigkeit eine Bewegung zu beginnen (freezing) und die Unmöglichkeit einer aufrechten Körperhaltung mit dem Risiko zu stürzen. Weiterhin wird das Denken, das Gedächtnis und die Kreislauffunktion beeinträchtigt sowie die Gefühle verändert mit Depressionen und im Endstadium Demenz.

Die Parkinson'sche Krankheit wird in eine sporadische Form (etwa 95 % der Betroffenen) und eine familiäre Form eingeteilt. Bei letzterer ist die Vererbung des Krankheitsrisikos die wichtigste Ursache. Außerdem sind viele Krankheiten beschrieben, bei denen Bewegungsstörungen auftreten, deren Entstehung aber auf anderen Ursachen beruhen. Diese werden als sekundärer Parkinsonismus bezeichnet. Diese Formen können durch Medikamente hervorgerufen werden wie z. B. Neuroleptika und Reserpin und seine Derivate. Außerdem ist ein Hemiatrophie-Hemiparkinson bekannt. Ein Parkinsonsyndrom ist auch beschrieben bei Hydrocephalus (Wasserkopf), Sauerstoffmangel, Infektionen des Gehirns (Encephalitis), Vergiftungen mit Mangan, Kohlenmonoxid (CO), 1-Methyl-4-phenyl-1,2,3,6-tetrahydropyridin (MPTP) und Cyanid. Weitere Ursachen sind Erkrankungen der Nebenschilddrüse, ein Tumor im Gehirn, eine Hirnverletzung und multiple Verschlüsse (Infarkte) von Hirngefäßen. Weitere Erkrankungen mit Bewegungsstörungen sind die Alzheimer-Krankheit, die diffuse Lewy-Körperchen Krankheit, die frontotemporale Demenz, die Lytico-Bodig Krankheit (Parkinsonismus-Demenz-amyotrophe Lateralsklerose), striatonigrale Degeneration, sporadische olivo-ponto-cerebelläre Degeneration, progressive pallidale Atrophie, fortschreitende supranukleäre Lähmung (progressive supranuclear palsy), Huntington'sche Krankheit, x-Chromosom verknüpfte Dystonie (Morbus Lubag), mitochondriale Zytopathie mit striataler Nekrose, Neuroakanthocytose und Wilsonsche Krankheit. Eine verminderte Funktion dopaminerger Nervenzellen ist auch an dem sogenannten restless legs Syndrom beteiligt.

Derzeit wird zum Ausgleich des Dopaminmangels die Dopaminvorstufe L-DOPA angewendet, sowie Medikamente eingesetzt, die die Andockstellen (Rezeptoren) von Dopamin direkt stimulieren, sowie Medikamente, die den Abbau von Dopamin hemmen. Jedoch zeigten sich bald Nebenwirkungen einer Langzeittherapie mit L-DOPA, welche von Dyskinesien (abnorme, unwillkürliche Bewegungen) über Dystonien (schmerzhafte Muskelkrämpfe) bis hin zu abrupt abwechselnden Bewegungs-Erstarrungs-Phasen reichten. Außerdem wurde erkannt, dass L-DOPA zu einer Beschleunigung der Zerstörung der Dopamin-haltigen Nervenzellen im Gehirn führen kann.

In Deutschland leiden 1 bis 2 % der über sechzigjährigen Personen an der Parkinson'schen Krankheit. Somit besteht der dringende Bedarf, Medikamente bereitzustellen, welche zur Behandlung von Morbus Parkinson und den anderen Bewegungsstörungen geeignet sind.

Der vorliegenden Erfindung liegt die Aufgabe zugrunde physiologisch gut verträgliche Substanzen sowie pharmazeutische Formulierungen bereitzustellen, welche zur Prophylaxe und Behandlung von Bewegungsstörungen eingesetzt werden können.

Diese Aufgabe wird durch die technische Lehre der unabhängigen Ansprüche gelöst. Weitere vorteilhafte Ausgestaltungen, Aspekte und Details der Erfindung ergeben sich aus den abhängigen Ansprüchen, der Beschreibung und den Beispielen.

### Beschreibung

Die Erfindung betrifft die Verwendung der Verbindungen der allgemeinen Formel (I) 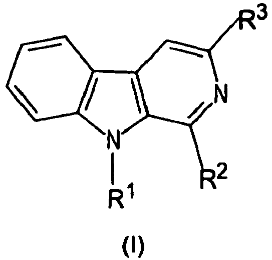 worin
**R¹** für einen der folgenden Reste steht:
   -R¹⁶, -CR⁴R⁵R⁶, -X-CR⁴R⁵R⁶, -X-R⁴, -CO-O-R¹⁶;
**R²** folgende Reste bedeutet: -H, -R⁷, -CR⁹R¹⁰R¹¹, -Y-CR⁹R¹⁰R¹¹, -Y-R¹⁵, -O-R¹⁷, -S-R¹⁸, -NH-R¹⁹, -CO-NH-R²⁰, -CO-O-R²¹;
**R³** folgende Reste bedeutet: -H, -R⁸, -Z-CR¹²R¹³R¹⁴, -Z-R¹⁵, -O-R¹⁷, -S-R¹⁸, -NH-R¹⁹, -CO-NH-R²⁰, -CO-O-R²¹;
**X** ausgewählt werden kann aus -(CH₂)ₙ-, -CH=CH-, -C=C-, -CH₂-CO-, -CO-, -CO-CH₂-;
**Y, Z** unabhängig voneinander bedeuten: -(CH₂)ₘ-, -CH=CH-, -C≡C-, -O-(CH₂)ₚ-, -NH-(CH₂)_{q}-, -NH-CO-, -CO-, -O-CO-;
m, p, q unabhängig voneinander eine ganze Zahl zwischen 1 und 4 darstellen;
n eine ganze Zahl zwischen 1 und 6 darstellt
**R⁴-R¹⁴** unabhängig voneinander folgende Reste bedeuten: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cylo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cylo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)_{2,} -NH-CO-N(C₂H₅)₂, -NH-CO-N(C₃H₇)₂, -NH-CO-N(cyclo-C₃H₅)₂, -NH-CO-N[CH(CH₃)₂]_{2,} -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cylo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cylo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃.
**R¹⁵** folgende Reste bedeutet: -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R⁴ - R⁷** und **R⁹ - R²¹** unabhängig voneinander folgende Reste bedeuten:
   -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
**R⁸** folgende Reste bedeutet:
   -CH₂F, -CHF₂, -CF₃, -CH₂Cl, -CH₂Br, -CH₂I, -CH₂-CH₂F, -CH₂-CHF₂, -CH₂CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂C(CH₃)₂-C₂H₅, -C(CH₃)₂C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂=CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₃, -CH₂-CH₂-CH₂-OCH₃, -C₂H₄-CH(CH₃)-CH=CH₂, -CH₂CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
sowie pharmakologisch verträgliche Salze, Solvate, Hydrate, Enantiomere, Diastereomere, Mischungen von Diastereomeren, Tautomere als auch Racemate der vorgenannten Verbindungen zur Herstellung eines Medikamentes für die Behandlung und/oder Prophylaxe von Bewegungsstörungen, Bewegungsstörungen aufgrund anderer Ursachen als der Parkinson'schen Krankheit, Alzheimer, Parkinson'schen Krankheit, Hemiatrophie-Hemiparkinson, Parkinsonsyndrom aufgrund von bzw. bei Hydrocephalus (Wasserkopf), Sauerstoffmangel, Infektionen des Gehirns (Encephalitis), Vergiftungen mit Mangan, Kohlenmonoxid (CO), 1-Methyl-4-Phenyl-1,2,3,6-Tetrahydropyridin (MPTP) und Cyanid, Erkrankungen der Nebenschildrüse, Hirnverletzung, Infarkte, sowie Lewy-Körperchen Krankheit, frontotemporale Demenz, Lytico-Bodig Krankheit (Parkinsonismus-Demenz-amyotrophe Lateralsklerose), striatonigrale Degeneration, Shy-Dräger-Syndrom, sporadische olivo-ponto-cerebelläre Degeneration, progressive pallidale Atrophie, fortschreitende supranukleäre Lähmung (progressive supranuclear palsy), Huntington'sche Krankheit, x-Chromosom verknüpfte Dystonie (Morbus Lubag), mitochondriale Zytopathie mit striataler Nekrose, Neuroakanthocytose, restless legs Syndrom und Wilson'sche Krankheit

Der hierin verwendete Ausdruck Tautomer ist als organische Substanz definiert, die durch eine chemische Reaktion, die Tautomerisierung, in ihr Gleichgewichtsisomer überführt werden kann. Die Tautomerisierung kann bevorzugt von Basen, Säuren oder anderen geeigneten Substanzen katalysiert werden.

### Allgemeine Synthese von 9-Alkyl-β-Carbolinen

Die Ausgangsverbindung Norharman kann nach literaturbekannten Vorschriften beispielsweise wie in Beispiel 9 beschrieben hergestellt werden.

Die N-Alkylierung in Position 9 erfolgt gemäß gängiger Alkylierungsreaktionen mittels Alkyliodiden, Alkylbromiden, Alkylchloriden, Alkylmesylaten, Alkyltosylaten oder anderen Alkylierungsreagenzien gemäß folgendem Reaktionsschema: 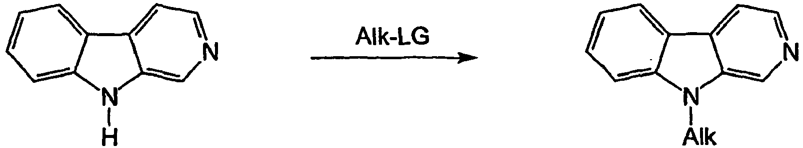 LG steht für eine Abgangsgruppe. Die Aklylierungsreaktionen sind vorzugsweise Basenkatalysiert.

### Allgemeine Reaktionsvorschrift zur Alkylierung:

1 mol Equivalent Norharman wird in einem getrockneten Lösungsmittel wie z.B. DMF, THF, Methylenchlorid, usw. unter Schutzgasatmosphäre gelöst. Die Deprotonierung erfolgt mittels eines Überschusses einer starken Base, vorzugsweise Natriumhydrd (ca. 2 mol Equivalente) bei erniedrigter Temperatur (0°C bis -78°C). Ebenfalls bei einer Temperatur unter 0°C wird 1,0 bis 1,2 mol Equivalente eines Alkylierungsmittels zugesetzt, welches in einem getrockneten Lösungsmittel gelöst sein kann. Man rührt die Mischung über Nacht, wobei sich die Reaktionslösung auf Raumtemperatur erwärmen kann. Die Aufarbeitung erfolgt in einer dem Fachmann bekannten Weise. Nicht umgesetztes Norharman kann durch eine lonenaustausch-Chromatographie oder Ionenpaar-Extraktion entfernt werden. Es ergeben sich in der Regel Ausbeuten von 30 bis 75% der Theorie.

Folgende Verbindungen wurden gemäß obiger Alkylierung hergestellt.

| | | |
|---|---|---|
| 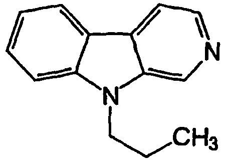 | 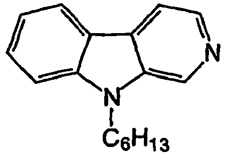 | 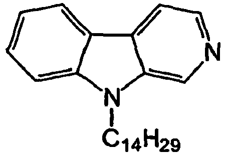 |
| Verbindung A | Verbindung B | Verbindung C |
| 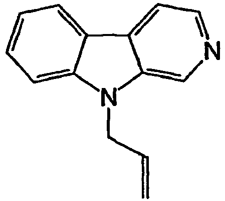 | 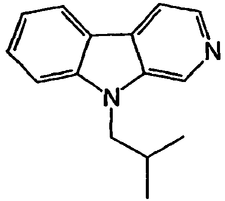 | 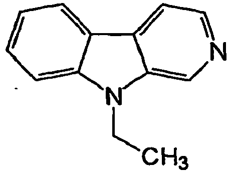 |
| Verbindung D | Verbindung E | Verbindung F |
| 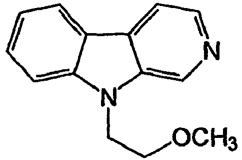 | 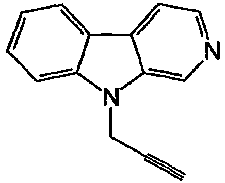 | 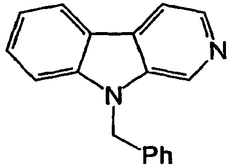 |
| Verbindung G | Verbindung H | Verbindung I |
| 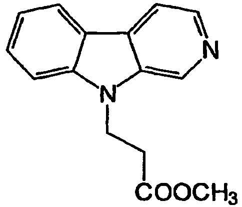 | 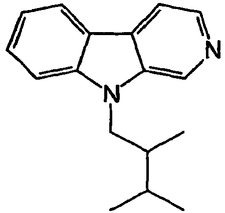 | 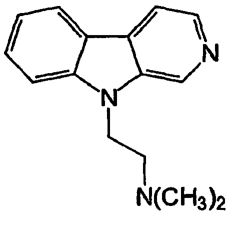 |
| Verbindung J | Verbindung K | Verbindung L |

### Allgemeine Synthese von 1,3-disubstituierten β-Carbolinen

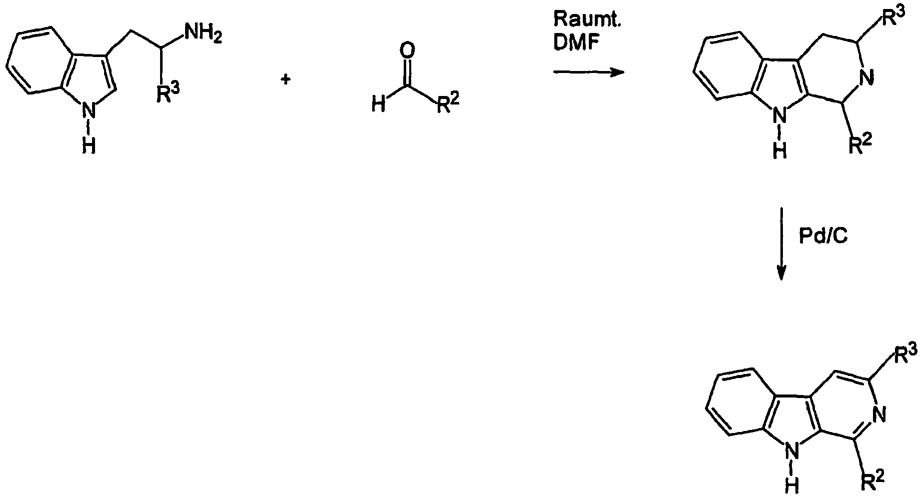

Um 1,3-disubstituierte β-Carboline zu erhalten, wurden Indolderivate mit den entsprechenden Aldehyden gemäß obigem Reaktionsschema umgesetzt.

Dabei wurden 0,1 Mol des Indolderivates in DMF gelöst und 0,12 Mol des Aldehyds unter Rühren hinzugefügt. Die Reaktionsmischung wurde 16 h bei Raumtemperatur gerührt. Nach Entfernen des Lösungsmittels wurde der erhaltene Feststoff zwei Mal in Toluol umkristallisiert und getrocknet. In einem weiteren Syntheseschritt wurden 0,07 Mol des aus Toluol umkristallisierten und getrockneten Feststoffes in 600 ml Cumol gelöst und unter Stickstoffatmosphäre mit 2,6 g Pd/C (10 %) für 90 min. Rückfluss erhitzt. Nach Zugabe von 100 ml Ethanol wurde die heiße Lösung filtriert und die Kohle mit 3 x 30 ml heißem Ethanol extrahiert. Die vereinigten flüssigen Fraktionen wurden vom unter Vakuum Lösungsmittel befreit und der Rückstand aus Toluol kristallisiert, um das 1,3-disubstituierte Norharman-Derivat zu erhalten.

Die entsprechenden 1-substituierten β-Carboline werden erhalten, wenn R³ ein Wasserstoffatom ist.

Die N-Alkylierung an Position 9 wird nach der Ringschlussreaktion mittels einer Base vorzugsweise eines Hydrids und anschließender Zugabe eines Alkylierungsmittels z.B. einer Alkyliodids vorgenommen. Eine ausführliche Vorschrift ist im experimentellen Teil enthalten. Gemäß dieser Reaktionsvorschrift wurden die folgenden Verbindungen hergestellt.

Bevorzugt sind folgende Verbindungen der allgemeinen Formeln (II) - (V)

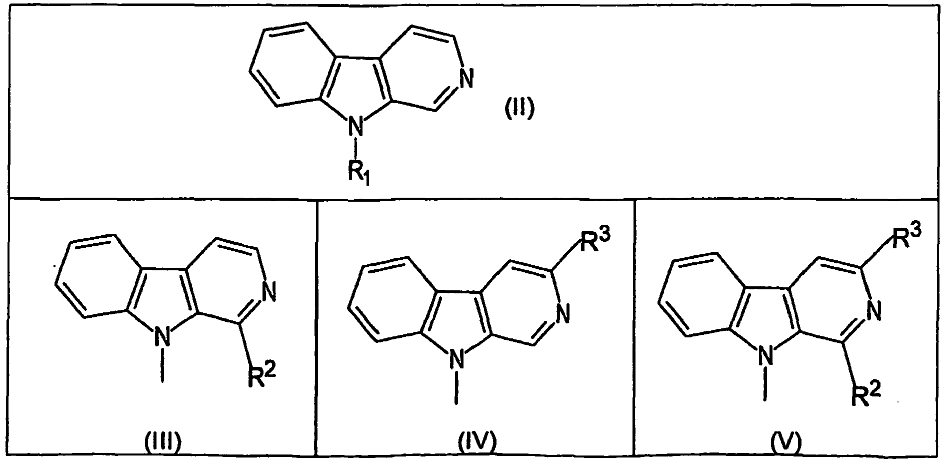

Die 9-Alkyl-β-Carboline zeigen überraschenderweise neuroprotektive Wirkung und fördern das Wachstum von neuen und funktionellen dopaminergen Nervenzellen.

Bevorzugt sind Substituenten an Positionen 9 (R¹), 1 (R²) und 3 (R³) des β-Carbolin-Ringsystems. Das Substitutionsmuster in R¹ umfasst bevorzugt Alkylsubstituenten, besonders bevorzugt den Methylsubstituenten. Die Reste R² und R³ umfassen besonders bevorzugt Alkylsubstituenten, Halogene und Alkoxysubstituenten.

Besonders bevorzugte Verbindungen sind demnach:

| | | |
|---|---|---|
| 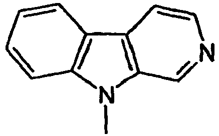 | 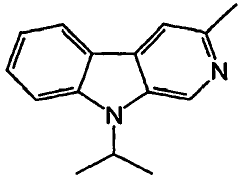 | 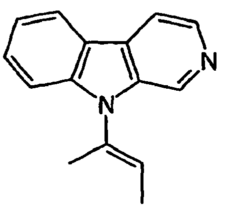 |
| 9-Methyl-9*H-*β-Carbolin (VI) | 9-iso-Propyl-9*H-*β-Carbolin (VII) | 9-[(1Z)-1-Methylprop-1-enyl]-9*H*-β-Carbolin (VIII) |
| 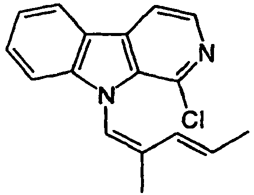 | 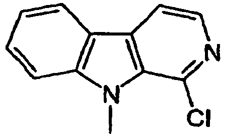 | 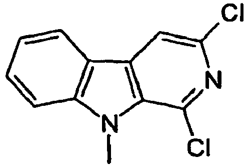 |
| 1-Chlor-9-[(1*Z*,3*E*)-2-Methylpenta-1,3-dienyl]-9*H*-β-Carbolin (IX) | 1-Chlor-9-Methyl-9*H-*β-Carbolin (X) | 1,3-Dichlor-9-Methyl-9*H*-β-Carbolin (XI) |
| 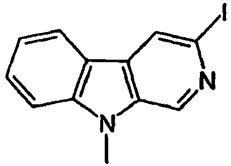 | 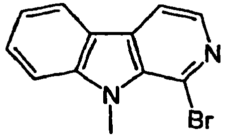 | 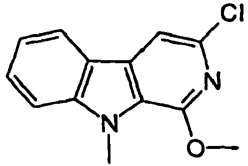 |
| 3-Iod-9-Methyl-9*H-*β-Carbolin (XII) | 1-Brom-9-Methyl-9*H*-β-Carbolin (XIII) | 1-Methoxy-3-Chlor-9-Methyl-9*H*-β-Carbolin (XIV) |
| 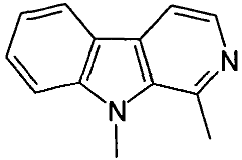 | 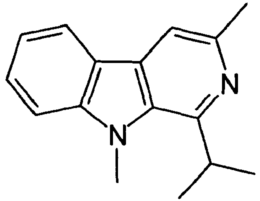 | 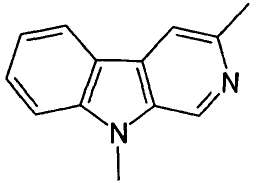 |
| 1,9-Dimethyl-9*H-*β-Carbolin (XV) | 1-Isopropyl-3,9-Dimethyl-9*H*-β-Carbolin (XVI) | 3,9-Dimethyl-9*H*-β-Carbolin (XVII) |
| 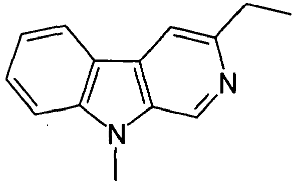 | 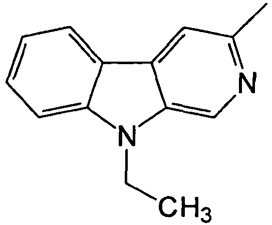 | 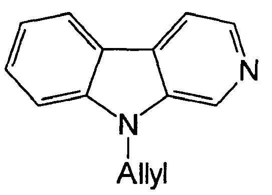 |
| 3-Ethyl-9-Methyl-9*H*-β-Carbolin (XVIII) | 9-Ethyl-3-methyl-9*H*-β-Carbolin (IXX) | 9-Allyl-3-isopropyl-9*H*-β-Carbolin (XX) |

Deshalb können die Verbindungen der vorliegenden Erfindung und insbesondere die bevorzugten Verbindungen der allgemeinen Formeln (II) - (XX) zur Herstellung einer pharmazeutischen Formulierung zur Behandlung und/oder Prophylaxe von Bewegungsstörungen, Bewegungsstörungen aufgrund anderer Ursachen als der Parkinson'schen Krankheit, Alzheimer, Parkinson'schen Krankheit, Hemiatrophie-Hemiparkinson, Parkinsonsyndrom aufgrund von bzw. bei Hydrocephalus (Wasserkopf), Sauerstoffmangel, Infektionen des Gehirns (Encephalitis), Vergiftungen mit Mangan, Kohlenmonoxid (CO), 1-Methyl-4-Phenyl-1,2,3,6-Tetrahydropyridin (MPTP) und Cyanid, Erkrankungen der Nebenschildrüse, Gehirntumor, Hirnverletzung, Infarkte, sowie Lewy-Körperchen Krankheit, frontotemporale Demenz, Lytico-Bodig Krankheit (Parkinsonismus-Demenzamyotrophe Lateralsklerose), striatonigrale Degeneration, Shy-Dräger-Syndrom, sporadische olivo-ponto-cerebelläre Degeneration, progressive pallidale Atrophie, fortschreitende supranukleäre Lähmung (progressive supranuclear palsy), Hallervorden-Spatz Erkrankung, Huntington'sche Krankheit, x-Chromosom verknüpfte Dystonie (Morbus Lubag), mitochondriale Zytopathie mit striataler Nekrose, Neuroakanthocytose und Wilson'sche Krankheit verwendet werden.

Unter dem Begriff Bewegungsstörungen sind insbesondere spastische Störungen, Hyperkinesien, Dystonien, Athetosen, Dyskinesien, Myoklonus-Syndrome, M. Wilson, choreatische Syndrome, Tics, Tourette-Störung, Ballismus, Tremor-Syndrome und Parkinson zu verstehen.

Die Verbindungen entsprechend der vorliegenden Erfindung können selbst oder in Form eines pharmakologisch wirksamen Salzes verabreicht werden. Da die Verbindungen vorliegenden Erfindung basische Eigenschaften besitzen können, können nach gängigen Methoden Salze dieser Verbindungen hergestellt werden.

Als Säuren, welche ein Säureadditionssalz mit den Verbindungen der vorliegenden Erfindung bilden, können die folgenden genannt werden: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-Mandelsäure, Hydrogenbenzolsulfonsäure, Pikrinsäure (2,4,6-Trinitrophenol), Adipinsäure, d-o-Tolylweinsäure, Aminosäuren wie Methionin, Tryptophan, Arginin und insbesondere saure Aminosäuren wie Glutaminsäure oder Asparaginsäure.

Je nach Art der Verbindung sind auch Betain-Formen möglich.

Ferner betrifft die vorliegende Erfindung pharmazeutische Zusammensetzungen, welche unter Verwendung mindestens einer erfindungsgemäßen Verbindung oder eines Salzes davon hergestellt wurden.

Neben mindestens einer Verbindung der vorliegenden Erfindung enthalten die pharmazeutischen Zusammensetzungen einen pharmakologisch verträglichen Träger, Hilfsstoff und/oder Lösungsmittel.

Die pharmazeutischen Zusammensetzungen können in Form von transdermalem Applikationssystem (Pflaster, Film), Tropfen, Mundspray, Nasenspray, Pillen, Tabletten, Filmtabletten, Schichttabletten, Zäpfchen, Gelen, Salben, Sirup, Inhalationspulvern, Granulaten, Suppositorien, Emulsionen, Dispersionen, Mikrokapseln, Kapseln, Puder oder Injektionslösungen hergestellt und verabreicht werden. Zudem Umfassen die erfindungsgemäßen pharmazeutischen Zusammensetzungen Formulierungen wie Schichttabletten zur kontrollierten und/oder kontinuierlichen Freisetzung des Wirkstoffs sowie Mikroverkapselungen als spezielle Darreichungsform.

Derartige Formulierungen sind unter anderem für die Inhalation oder die intravenöse, intraperitoneale, intramuskuläre, subkutane, mucokutane, orale, rektale, transdermale, topikale, bukkale, intradermale, intragastrale, intrakutane, intranasale, intrabuccale, perkutane oder sublinguale Verabreichung geeignet.

Als pharmakologisch verträgliche Träger können beispielsweise Lactose, Stärke, Sorbitol, Sucrose, Cellulose, Magnesiumstearat, Dicalciumphosphat, Calciumsulfat, Talk, Mannitol, Ethylalcohol und dergleichen eingesetzt werden. Puder als auch Tabletten können zu 5 bis 95% aus einem derartigen Träger bestehen.

Als Bindemittel können zudem Stärke, Gelatine, natürliche Zucker, sowohl natürliche als auch synthetische Gummis wie beispielsweise Akaziengummi oder Guar-Gummi, Natriumalginat, Carboxymethyl-Cellulose, Polyethylenglycol und Wachse eingesetzt werden. Als Gleitmittel können Borsäure, Natriumbenzoat, Natriumacetat, Natriumchlorid und dergleichen dienen.

Ferner können den pharmazeutischen Zusammensetzungen noch Sprengmittel, Farbstoffe, Geschmacksstoffe und/oder Bindemittel zugesetzt werden.

Flüssige Formulierungen umfassen Lösungen, Suspensionen, Sprays und Emulsionen. Beispielsweise Injektionslösungen auf Wasserbasis oder Wasser-Propylenglycol-Basis für parenterale Injektionen.

Für die Zubereitung von Suppositorien werden bevorzugt niedrigschmelzende Wachse, Fettsäureester und Glyceride eingesetzt.

Kapseln werden beispielsweise aus Methylcellulose, Polyvinylalkohole oder denaturierte Gelatine oder Stärke hergestellt.

Als Sprengmittel können Stärke, Natrium-Carboxymethylstärke, natürliche und synthetische Gummis wie beispielsweise Johannisbrotkemmehl, Karaya, Guar, Tragacanth und Agar sowie Cellulosederivate wie Methylcellulose, Natrium-Carboxymethylcellulose, microkristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Korn, Reis oder Kartoffeln, natürliche Gummis wie Akaziengummi, Gelatine, Tragacanth, Alginsäure, Natriumalginat, Ammonium-Calcium-Alginat, Methylcellulose, Natrium-Carboxymethylcellulose, Hydroxypropyl-methylcellulose, Polyvinylpyrrolidon sowie anorganische Verbindungen wie Magnesium-Aluminum-Silicate zugesetzt werden. Die Bindemittel könne in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol und Aminosäuren wie Leucin eingesetzt werden. Derartige Gleitmittel können in Mengen von 0,05 bis 15 Gew.-% verwendet werden.

Somit sind die hier vorgestellten Verbindungen nützlich für die Herstellung von pharmazeutischen Formulierungen zur Behandlung von Morbus Parkinson und anderen Bewegungsstörungen.

Ein weiterer Aspekt der vorliegenden Erfindung ist auf die Verwendung der Verbindungen gemäß allgemeiner Formel (I) 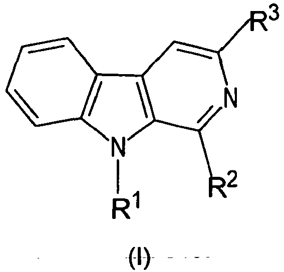 worin
**R¹** für einen der folgenden Reste steht:
   -R¹⁶, -CR⁴R⁵R⁶, -X-CR⁴R⁵R⁶, -X-R⁴, -CO-O-R¹⁶;
**R²** folgende Reste bedeutet: -H, -R⁷, -CR⁹R¹⁰R¹¹, -Y-CR⁹R¹⁰R¹¹, -Y-R¹⁵, -O-R¹⁷, -S-R¹⁸, -NH-R¹⁹, -CO-NH-R²⁰, -CO-O-R²¹;
**R³** folgende Reste bedeutet: -Z-CR¹²R¹³R¹⁴, -O-R¹⁷, -S-R¹⁸, -NH-R¹⁹, -CO-NH-R²⁰;
**X** ausgewählt werden kann aus -(CH₂)ₙ-, -CH=CH-, -C≡C-, -CH₂-CO-, -CO-, -CO-CH₂-;
**Y, Z** unabhängig voneinander bedeuten: -(CH₂)ₘ-, -CH=CH-, -C≡C-, -O-(CH₂)ₚ-, -NH-(CH₂)_{q}-, -NH-CO-, -CO-, -O-CO-;
m, p, q unabhängig voneinander eine ganze Zahl zwischen 1 und 4 darstellen;
n eine ganze Zahl zwischen 1 und 6 darstellt
**R⁴ - R¹⁴** unabhängig voneinander folgende Reste bedeuten: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R¹⁵** folgende Reste bedeutet: -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R⁴ - R⁷** und **R⁹ - R²⁰** unabhängig voneinander folgende Reste bedeuten:
   -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, *-C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
**R⁸** folgende Reste bedeutet:
   -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H,₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅ -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH3)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃; sowie pharmakologisch verträgliche Salze, Solvate, Hydrate, Enantiomere, Diastereomere als auch Racemate der vorgenannten Verbindungen zur Beschleunigung des Wachstums von Zellen insbesondere in (Zell)Kulturmedien, Nährmedien und Fermentationsmedien gerichtet. Dazu wird mindestens eine Verbindung gemäß allgemeiner Formel (I) oder ein physiologisch verträgliches Salz davon dem Medium in einer Konzentration von 0,5 bis 500 µM, vorzugsweise 1,0 bis 250,0 µM, weiter bevorzugt 10,0 µM bis 100,0 µM und insbesondere bevorzugt 40,0 µM bis 60,0 µM als Additivum zugesetzt.

Ferner ist die vorliegende Erfindung auf (Zell)Kulturmedien, Nährmedien und Fermentationsmedien gerichtet, welche mindestens eine Verbindung gemäß allgemeiner Formel (I) enthalten und dies vorzugsweise in den oben angegebenen Konzentrationsbereichen.

Der wachstumsbeschleunigende Effekt der Verbindungen gemäß allgemeiner Formel (I) konnten bei vielen Zelltypen sowie in humanen als auch tierischen Zellkulturen nachgewiesen werden. Bevorzugte Zellen sind Knochenzellen, Knorpelzellen, Fibroblasten, neuronale Zellen, Gliazellen, dopaminerge Zellen, Fettzellen, Muskelzellen, Herzmuskelzellen, Hautzellen, Hepatozyten, Mesenchymzellen, gonadotropen Zellen, Cumuluszellen, Blutzellen, Epithelzellen, Endothelzellen, Basalzellen, adulte und embryonale Stammzellen und multipotente Vorläuferzellen.

### Beschreibung der Figuren

- Fig. 1a: Dosisabhängige Inkubation dopaminerger Nervenzellen für 48 Stunden (Kontrolle).
- Fig. 1b: Dosisabhängige Inkubation dopaminerger Nervenzellen mit 50 µM 9-Methyl-β-Carbolin für 48 Stunden (DIV=Days in vitro).
- Fig.1c: Prozentsatz dopaminerger Nervenzellen (Dopamin-Neuronen) nach Inkubation mit 10, 25, 50, 100, 200 µM 9-Methyl-β-Carbolin für 48 Stunden von 10 DIV - 12 DIV.
- Fig.1d: Prozentsatz dopaminerger Nervenzellen nach Ausschaltung des Dopamintransporters DAT durch GBR12909 nach 48 Stunden. Einfluss der DAT-Inhibition durch GBR12909 auf die Wirkung von 50 µM 9-Me-BC auf die Anzahl von TH-positiven Neuronen während eines 48-stündigen Inkubationszeitraumes.
- Fig. 2a: Dopaminkonzentration (Dopamin-Gehalt) nach Inkubation mit 50 µM 9-Methyl-β-Carbolin für 48 Stunden von 10 DIV -12 DIV.
- Fig. 2b: Dopamin-Transportkapazität dopaminerger Nervenzellen nach 48 Stunden. ³H-Dopamin-Aufnahme nach Inkubation mit 50 µM 9-Me-BC für 48 Stunden von 10 DIV-12 DIV.
- Fig. 3: Prozentsatz der Nervenzellen (gesamt) nach Inkubation 50 µM 9-Methyl-β-Carbolin für 48 Stunden von 10 DIV -12 DIV.
- Fig. 4a: LDH-Abgabe nach 48 Stunden Inkubation mit 50 µM 9-Methyl-β-Carbolin für 48 Stunden 10 DIV - 12 DIV.
- Fig. 4b: Prozentsatz abgestorbener (nekrotischer) Zellen nach Inkubation mit 50 µM 9-Methyl-β-Carbolin für 48 Stunden 10 DIV -12 DIV.
- Fig. 4c: ATP-Konzentration (ATP-Gehalt) nach Inkubation mit 50 µM 9-Methyl-β-Carbolin (9-Me-BV) für 48 Stunden 10 DIV -12 DIV.
- Fig. 4d: Aktivität des Apoptose-Enzyms Caspase 3 während und nach Inkubation mit 50 µM 9-Methyl-β-Carbolin für 48 Stunden 10 DIV -12 DIV.
- Fig. 5: Prozentsatz von BrdU-positiven Zellen humaner Neuroblastomzellen nach Inkubation mit 50 µM 9-Methyl-β-Carbolin für 48 Stunden 10 DIV -12 DIV.
- Fig. 6: Nachweis der Verminderung der Zellproliferation durch Einbau von Bromdesoxyuridin in das Zellkern-Chromatin.
- Fig. 7: Nachweis der Genexpresison von neurotrophischen Trannskriptionsfaktoren und Dopamin-Markern mit Real-Time-RT-PCR nach Inkubation mit 50 µM 9-Methyl-β-Carbolin für 48 Stunden 10 DIV -12 DIV.
- Fig. 8: zeigt die Dopamin-Spiegel (ng / mg Gewebe) im Striatum der Ratte nach 6-wöchiger Infusion in den linken Ventrikel des Gehirns der Ratte von anfangs MPP+ (0,284 mg / kg / Tag) für die ersten 4 Wochen und für die letzten beiden Wochen NaJ als Vehikel (Natriumiodid: 0,0716 mg / kg / Tag). Angegeben in dem Diagramm sind die Dopamin (DA) Spiegel des linken und rechten Striatums der einzelnen Ratten.
- Fig. 9: zeigt die Dopamin-Spiegel (ng / mg Gewebe) im Striatum der Ratte nach 6-wöchiger Infusion in den linken Ventrikel des Gehirns der Ratte von anfangs MPP+ (0,284 mg / kg / Tag) für die ersten 4 Wochen und für die letzten beiden Wochen 9-Methyl-Betacarbolin (0,105 mg / kg / Tag).

### Beispiele

### Beispiel 1

Zum Nachweis der Wirksamkeit der erfindungsgemäßen 9-Alkyl-β-Carboline wurden Versuche durchgeführt, um festzustellen, welche Auswirkungen diese Verbindungsklasse auf die Bildung von neuen Dopamin-enthaltenden Nervenzellen hat. Als Beispielsubstanz wurde 9-Methyl-β-Carbolin (9-Me-BC) ausgewählt.

Die Versuche wurden mit der humanen Neuroblastom-Zelllinie (SH-SY5Y-Zellen) und mit einer Zellkultur primärer Nervenzellen des Mittelhirns von Mäuseembryonen durchgeführt. Dabei wurde festgestellt, dass 9-Alkyl-β-Carbolin-Derivate neuroprotektiv wirken. Außerdem induzieren die erwähnten Verbindungen in der Primärkultur des Mittelhirns von Mäuseembryonen das Auftreten von neuen, differenzierten und funktionellen dopaminergen Nervenzellen.

Hierfür wurden Mittelhimkulturen präpariert und 10-12 Tage (DIV=Days in vitro) in einem geeigneten Medium gehalten, um sie an die *in vitro*-Bedingungen zu gewöhnen. Dann wurde 9-Methyl-β-Carbolin in ansteigenden Konzentrationen zugegeben und 48 Stunden gewartet. Die dopaminergen Nervenzellen wurden durch Anfärben von Tyrosinhydroxylase identifiziert. Dieses Enzym kommt im Mittelhim nur in dopaminergen Nervenzellen vor. Die Zahl der dopaminergen Nervenzellen kann mit dieser Methode zuverlässig gezählt werden. Außerdem können Hinweise dafür gewonnen werden, ob die dopaminergen Nervenzellen reif sind oder ob es sich um Vorstufen handelt. Die reifen Zellen haben charakteristische Ausläufer, die ebenfalls Tyrosinhydroxylase enthalten.

### Ergebnisse:

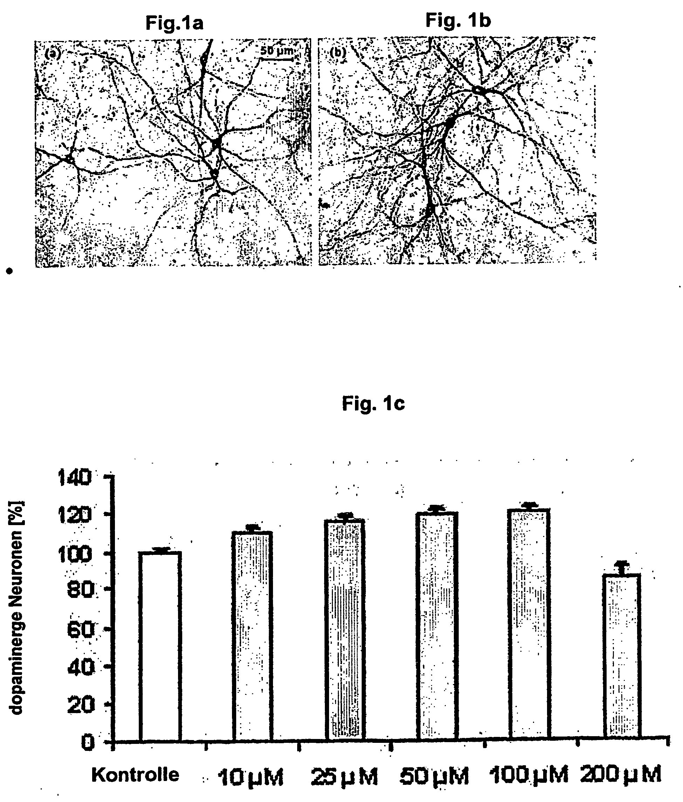

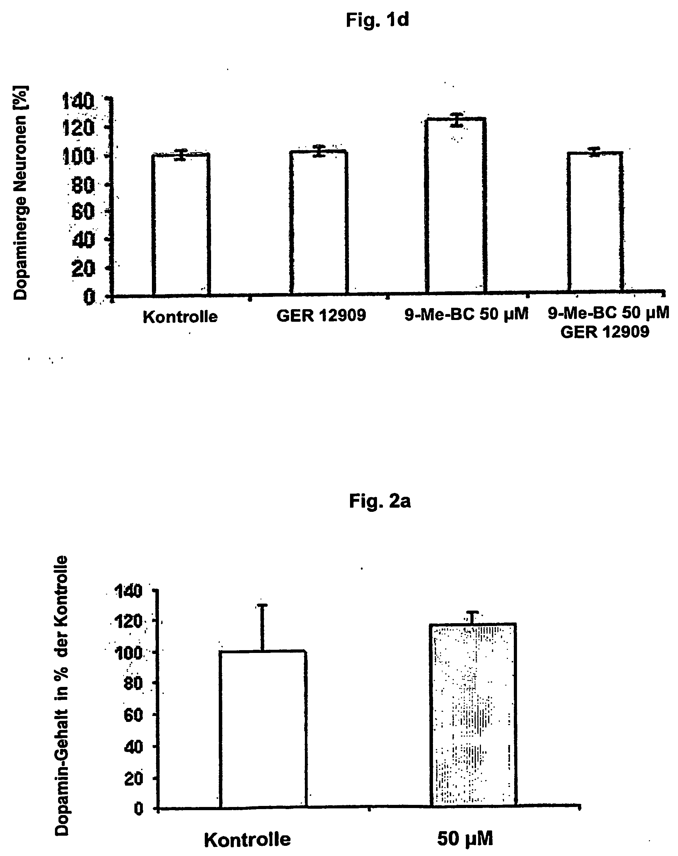

Die dopaminergen Nervenzellen, die 9-Methyl-β-Carbolin für 48 Stunden ausgesetzt wurden, hatten deutlich mehr Zellfortsätze und diese waren deutlich häufiger verzweigt als die Nervenzellen ohne 9-Methyl-β-Carbolin (Fig. 1 a: Kontrolle, Fig. 1 b: 50 µM 9-Methyl-β-Carbolin). Außerdem nahm die Zahl der dopaminergen Nervenzellen bis zu einer Konzentration von etwa 100 µM (Fig. 1 c) dosisabhängig zu. Die Wirkung von 9-Methyl-β-Carbolin benötigt eine intakte Funktion des sogenannten Dopamintransporters (DAT). Bei einer selektiven Ausschaltung des Transporters z.B. durch GBR12909 bleibt die Zunahme der dopaminergen Nervenzellen aus (Fig. 1d). 9-Methyl-β-Carbolin wird also durch den DAT in die dopaminergen Nervenzellen hineintransportiert und kann erst dann seine Wirkung entfalten.

### Beispiel 2

Um weitere Hinweise darauf zu erhalten, dass tatsächlich die Zahl der dopaminergen Nervenzellen zugenommen hat, wurde die Konzentration von Dopamin gemessen. Sie stieg leicht an, ohne dass der Anstieg Signifikanzniveau erreichte (Fig. 2a). Eine weitere Besonderheit und Spezifität von dopaminergen Nervenzellen ist der Transport von Dopamin in die Nervenzellen hinein. Dopamin kann wegen seines hydrophilen Charakters die Außenmembran von Nervenzellen nicht durchdringen. Es muss aktiv und energieabhängig durch den DAT-Mechanismus transportiert werden.

### Ergebnisse:

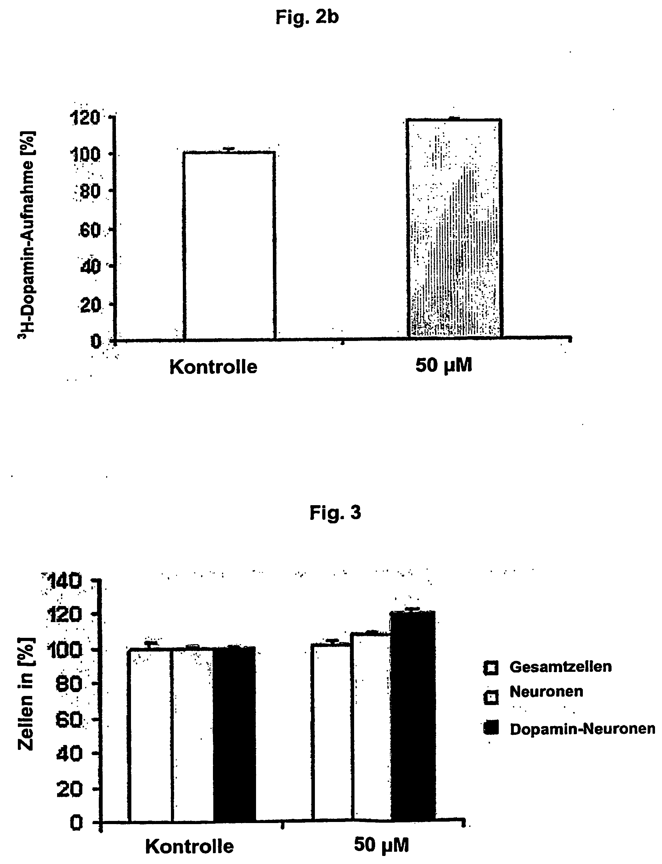

Die Transportkapazität der Nervenzellen für Dopamin nimmt in der Kultur, die 9-Methyl-β-Carbolin enthält, zu (Fig. 2b). Dies ist ein starker Hinweis auf eine Vermehrung von funktionellen, dopaminergen Nervenzellen.

### Beispiel 3

9-Methyl-β-Carbolin wurde verwendet, um zu zeigen, ob nur dopaminerge Nervenzellen beeinflusst werden oder auch andere Nervenzellen oder Gliazellen. Diese Frage kann durch Einsatz verschiedener Farbstoffe beantwortet werden. Wie Fig. 3 zeigt, nimmt die Gesamtzahl der Nervenzellen zu, jedoch nicht so stark wir die der dopaminergen Nervenzellen. Dopaminerge Nervenzellen sind also empfindlicher gegenüber den Wirkungen von 9-Methyl-β-Carbolin. Dies schließt aber nicht aus, dass eine längere Exposition zu einer signifikanten Vermehrung auch anderer Nervenzelltypen führt.

### Beispiel 4

Es wurden Experimente an Mittelhirnzellen in Kultur mit der Fragestellung durchgeführt, ob 9-Methyl-β-Carbolin allgemein neuroprotektiv wirkt.

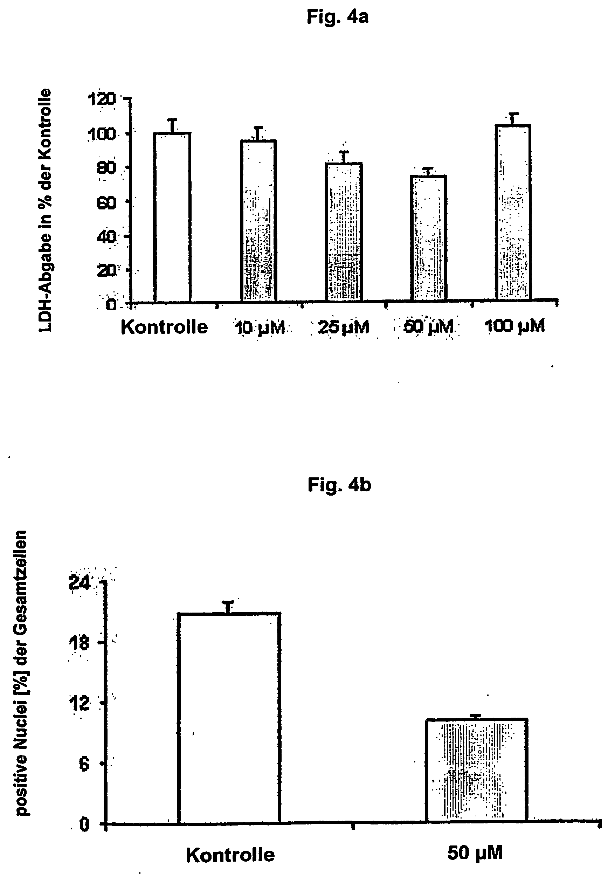

Absterbende Zellen setzen das Enzym Lactatdehydrogenase (LDH) in das Medium frei. Da in Kultur immer Zellen absterben, ist immer eine gewisse Konzentration an LDH im Medium nachweisbar, als ein indirektes Maß für die Vitalität der Kultur. Verglichen mit Kontrollbedingungen nahm die LDH Konzentration nach Inkubation mit 9-Methyl-β-Carbolin dosisabhängig bis zu einer Konzentration von 50 µM ab (Fig. 4a). Die Zahl der abgestorbenen Zellen kann durch Anfärbung der Zellkerne mit Propidiumiodid quantifiziert werden.

### Ergebnisse

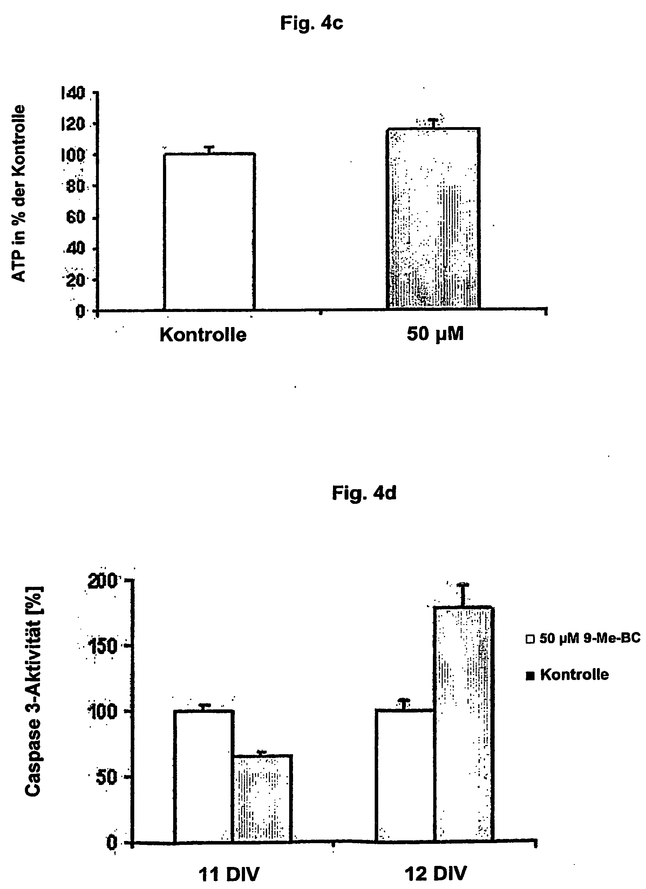

In 48 Stunden sterben etwa 20 % der Zellen in Kultur ab. In Kulturen mit einem 9-Alkyl-β-Carbolin starben unter denselben Bedingungen nur etwa 10 % der Zellen ab (Fig. 4b). Die Befunde gehen also in die gleiche Richtung wie die LDH-Befunde. ATP stellt der Zelle Energie zur Verfügung. Interessanterweise stieg die Konzentration an ATP in der Zellkutur mit einem 9-Alkyl-β-Carbolin an (Fig. 4c). Das Enzym Caspase 3 ist ein Indikator für den programmierten Zelltod (Apoptose). Nach 24 Stunden war die Aktivität des Enzyms vermindert, was für eine Verminderung apoptotischer Vorgänge spricht, nach 48 Stunden jedoch erhöht (Fig. 4d). Der letzte Befund spricht für eine Umstellung der Kulturzellen von einer verminderten Nekrose auf Apoptose, also eine genauere Regulierung des Zelltods.

### Beispiel 5

Die Frage, ob 9-Alkyl-β-Carbolin-Derivate zur Differenzierung von Nervenzellen führen, ist von eminenter Bedeutung. Sie wurde deshalb auch an einem anderen Modell nämlich an humanen Neuroblastomzellen untersucht.

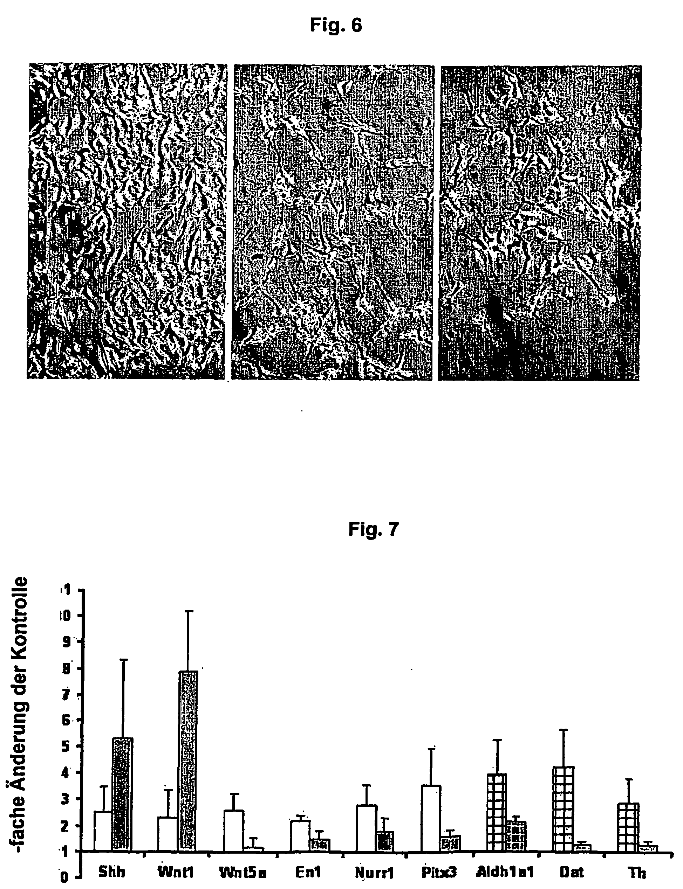

Diese Zelllinie hat viele Eigenschaften, die auch dopaminerge Neurone haben. Exposition der Zellen mit 9-Methyl-β-Carbolin führte zu einem Auswachsen von Zellfortsätzen (Fig. 6). Dies bestätigt also die Beobachtungen in primären Mittelhimkulturen, dass 9-Methyl-β-Carbolin die Differenzierung und damit Reifung neuronaler Zellen fördert (Fig. 1a und Fig. 1b). Aus der Fig. 6 geht auch hervor, dass die mit 9-Methyl-β-Carbolin exponierten Zellen sich weniger vermehren (proliferieren) als die Kontrollzellen.

### Ergebnisse

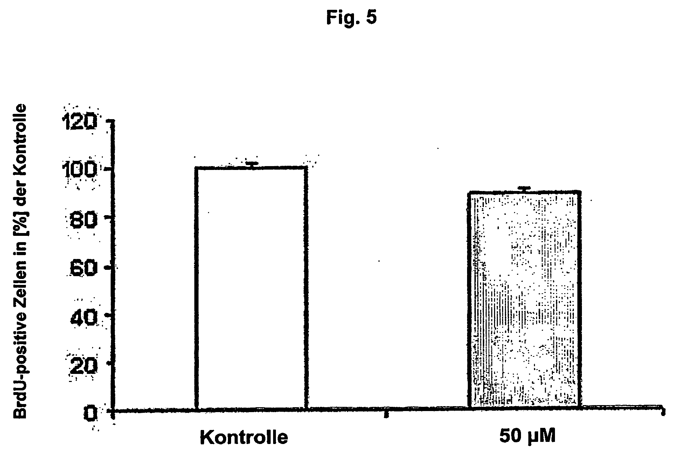

Die meisten Beobachtungen zeigen, dass Proliferation und Differenzierung sich gegenseitig mehr oder weniger ausschließen. Die Proliferation von Zellen kann mit dem Einbau von Bromdesoxyuridin gemessen werden. Wie Fig. 5 zeigt, nimmt der Einbau von Bromdeoxyuridin in das Chromatin im Zellkern ab. Dies spricht für eine Verminderung der Zellproliferation. Auch dieser Befund unterstützt die anderen Befunde, dass 9-Alkyl-β-Carbolin-Derivate die Differenzierung von Zellen fördert.

### Beispiel 6

In einer weiteren Serie von Experimenten wurde untersucht, wie die Zunahme der dopaminergen Nervenzellen zustande kommt.

Des weiteren, kann die Zahl der abgestorbenen Zellen durch Anfärbung der Zellkerne mit Propidiumiodid direkt quantifiziert werden.

Dieser Vorgang ist bisher noch nach keiner Substanz beobachtet worden, schon gar nicht nach einer Inkubationszeit von nur 48 Stunden. Die Bedeutung der Beobachtung wird auch dadurch unterstrichen, dass auch nach Absetzten von 9-Alkyl-β-Carbolinen die Zahl der dopaminergen Nervenzellen erhöht bleibt.

Dazu wurden Real-Time-RT-PCR-Experimente durchgeführt. Mit dieser quantitativen Methode wird die Transkription von ausgewählten Genen gemessen. Wir wählten Gene aus, die unter Proliferations- und Differenzierungsbedingungen erhöht sind sowie solche, die für die Bildung, Stabilität und den Abbau von dopaminergen Nervenzellen von Bedeutung sind. Die Analysen wurden an Mittelhirnkulturen nach Exposition mit 50 µM 9-Methyl-β-Carbolin für 48 Stunden und 48 Stunden nach Absetzen von 9-Methyl-β-Carbolin durchgeführt. Kontrollkulturen wurden simultan unter den gleichen Bedingungen gehalten. Die Ergebnisse sind aus Fig. 7 ersichtlich. Diese Untersuchungen ergaben, dass in Kulturen mit 9-Methyl-β-Carbolin solche Faktoren aktiviert waren, die die Differenzierung von Vorläuferzellen in neuronale Zellen stimulieren (Sonic Hedgehog) und die die Bildung von Tyrosinhydroxylase-positiven Nervenzellen (dopaminerge Nervenzellen) aktivieren (Wnt 1, Wnt 5a, engrailed 1, Nurr 1, und Pitx 3). Außerdem waren Faktoren erhöht, die bei der Bildung von dopaminergen Vorstufen (Progenitorzellen) aktiviert sind (Aldh1a1), sowie der Dopamintransporter (DAT) und die Tyrosinhydroxylase.

Diese Befunde wurden durch sogenannte Microarray-Methoden weiter abgesichert. Die Analysen bestätigten, dass Gene, die an der Differenzierung von Nervenzellen beteiligt sind, vermehrt transkribiert werden. Interessanterweise wurden Gene, die Entzündungsprozesse aktivieren, geringer transkribiert als in Kontrollkulturen. Gene, die an Apoptose beteiligt sind, wurden weniger transkribiert.

### Ergebnisse

Insgesamt zeigen die Analysen, dass 9-Alkyl-β-Carbolin-Derivate dopaminerge Nervenzellen induzieren und stabilisieren und dass die 9-Alkyl-β-Carbolin-Derivate eine allgemeine zellprotektive Wirkung besitzen, die auch für andere Typen von Nervenzellen günstig sind. Diese Wirkungen haben ein erhebliches therapeutisches Potential für die Behandlung von neurodegenerativen Erkrankungen insbesondere die Parkinson'sche Krankheit und den Morbus Alzheimer sowie von Bewegungsstörungen.

### Beispiel 7

Nachweis des wachstumsbeschleunigenden Effekts auf Zellen

Die Zellwachstums-steigernde Wirkung der Verbindungen gemäß allgemeiner Formel (I) kann insbesondere bei der Kultivierung von dopaminergen Zellen und auch bei Stammzellen genutzt werden, die zur Behandlung der Parkinson'schen Erkrankung transplantiert werden sollen.

Um zu prüfen, ob 9-Methyl-β-Carbolin die Effizienz von Kulturmedien für die Untersuchung von Nervenzellen und für das tissue engineering erhöht, wurden Pilot-Experimente exemplarisch mit undifferenzierten, multipotenten, neuronalen Vorläuferzellen aus dem Mittelhirn von Mäuseembryonen (Embryonaltag 14) durchgeführt. Zur Kultivierung der Zellen wurde durch geeignete Verfahren eine Zellsuspension hergestellt, die dann vier Wochen in einem Medium gehalten wurde, das u.a. Wachstumsfaktoren aus Fibroblasten und der Haut (epidermal growth factor) enthielten. Danach wurden die neuronalen Vorläuferzellen in einem Medium zur Förderung der Reifung von Nervenzellen differenziert. Nach einer Woche konnten etwa 40 % der Zellen mit einem Marker für Nervenzellen angefärbt werden (ß-Tubulin, Typ III). Die meisten anderen Zellen waren Gliazellen. Ein geringer Prozentsatz der Nervenzellen konnte zusätzlich mit dem Antikörper für Tyrosinhydroxylase angefärbt werden (∼3%). Die bedeutet, dass etwa drei Prozent der Nervenzellen vom dopaminergen Zelltyp waren. Das Enzym Tyrosinhydroxylase kommt im Mittelhirn nur in dopaminergen Nervenzellen vor. Um diesen Befund noch weiter zu sichern, wurde mit der Methode der real-time RT-PCR nachgewiesen, dass die Kultur ausser Tyrosinhydroxylase noch andere Proteine enthält, die charakteristisch für dopaminerge Nervenzellen sind, nämlich den Dopamintransporter DAT und die Aldehyddehydrogenase 1A1 (ALDH 1A1).
Unter entsprechenden Bedingungen wurden dem Nährmedium 9-Methyl-ß-Carbolin (50µM Endkonzentration) einmal pro Woche zugefügt und auch einmal dem Differenzierungsmedium. Die Menge an Nervenzellen stieg im Vergleich zu den Kontrollbedingungen deutlich an (+ 15 %, 3 unabhängige Experimente), während der Anteil an dopaminergen Zellen sogar um 25 % anstieg. Diese Ergebnisse belegen eindeutig, dass die Verbindungen gemäß allgemeiner Formel (I) am Beispiel von 9-Methyl-ß-Carbolin die Neubildung von Nervenzellen anregen und die Vorläuferzellen sich bevorzugt in dopaminerge Nervenzellen entwickeln. Die Verbindungen gemäß allgemeiner Formel (I) sind also geeignet, um neuronale Differenzierung ganz allgemein zu studieren und speziell eine Kultur mit einem retativ hohen Anteil an dopaminergen Nervenzellen zu gewinnen. Dies ist beispielsweise für die Implantation bei Patienten mit der Parkinson'schen Erkrankung, bei der vorwiegend dopaminerge Nervenzellen zugrunde gegangen sind, von grossem Interesse. Ausserdem sind die Verbindungen gemäß allgemeiner Formel (I) geeignet für tissue engineering, bei dem bestimmte Typen von Zellen und insbesondere . Nervenzellen (z. B. hippokampale Kömerzellen) gezielt vermehrt werden sollen. Die Anwendung von Verbindungen gemäß allgemeiner Formel (I) in Zellkulturen führt zu einer Beschleunigung des Zellwachstums, was insbesondere bei Nervenzellen, Knochenzellen, Stammzellen, Endothelzellen und anderen zur Implantation vorgesehener Zellen von großem Interesse ist und die Behandlung von Leukämie oder neurodegenerativer Erkrankungen deutlich verbessert.

### Beispiel 8

Um die Parkinson'sche Erkrankung zu untersuchen, werden seit Jahren akute in vivo Modelle eingesetzt, obgleich chronische Modelle die Erkrankung besser darstellen würden. Ein weiterer Nachteil der akuten Modelle ist es, dass ca. 40 % der Tiere an den Folgen der Applikation des Neurotoxins sterben. Wir haben deshalb ein chronisches Modell gewählt, bei dem vergleichsweise sehr niedrige Dosen des Neurotoxins 1-Methyl-4-Phenylpyridinium (MPP⁺) 28 Tage lang in den linken Seitenventrikel des Rattengehirns infundiert wurden. Die Substanz wurde durch osmotische Minipumpen kontinuierlich appliziert. Dadurch wurden Todesfälle vollständig vermieden. Nach vier Wochen wurde über denselben Katheter entweder Lösungsmittel oder äquimolare Mengen der Testsubstanz, nämlich das neuroprotektiv wirkende Betacarbolin bzw. 9-Alkyl-Betacarbolin, zwei Wochen lang infundiert. In einer Vorabstudie wurde in Dosisfindungsexperimenten die MPP⁺ Dosis gefunden, die zu einer Absenkung der Dopaminkonzentration im Striatum um etwa 40 % führt. Die Dopaminkonzentration im Striatum ist ein charakteristischer Marker für die Parkinson'sche Erkrankung, bei der es zum Untergang der Dopaminhaltigen Nervenzellen kommt.

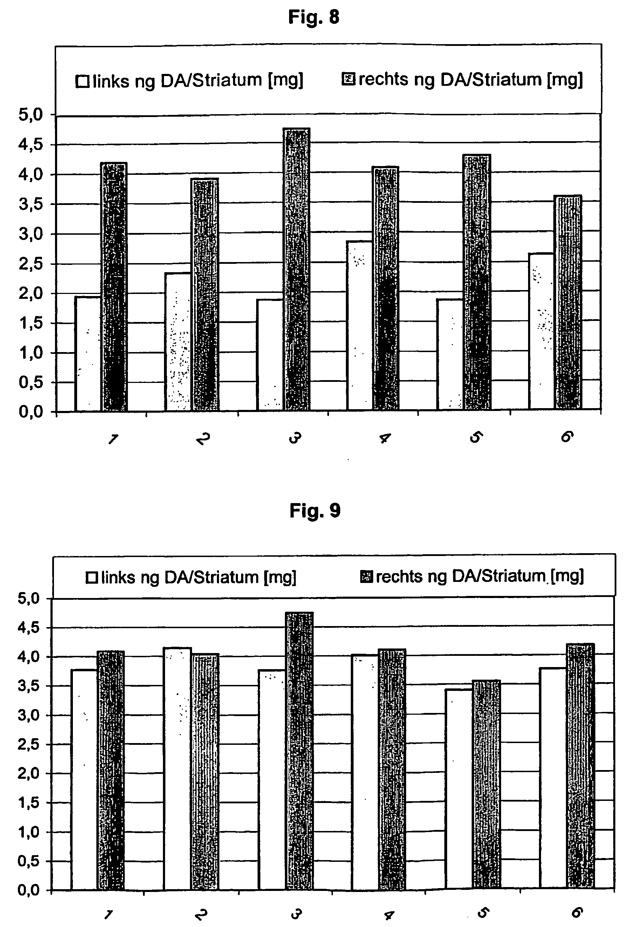

Die Ergebnisse sind in Figur 8 und 9 dargestellt. Es zeigte sich, dass die Dopaminkonzentration nur auf der linken Seite abnimmt, in die das Neurotoxin MPP⁺ infundiert worden war, während die Konzentration auf der rechten Seite der von Kontrollratten entsprach. Im Gegensatz dazu normalisierte das Betacarbolin bzw. 9-Alkyl-Betacarbolin die Konzentration der linken Seite, wenn es während der zwei Wochen nach dem MPP⁺ in äquimolaren Konzentrationen infundiert worden war.

Eine Erklärung für die Wirkung des Betacarbolins wurde in Untersuchungen der RNA aus den Striata dieser Ratten mittels der PCR-Superarray Methode gefunden. Danach aktiviert das Betacarbolin die Bildung von Neurotrophinen, also körpereigenen Proteinen, die für das Wachstum und die Differenzierung von Nervenzellen verantwortlich sind. Beispielsweise stieg der Brain-derived neurotrophic factor (BDNF) um das 4,9-fache im linken Striatum an im Vergleich zu Ratten, denen nur Lösungsmittel sechs Wochen lang infundiert worden war. Bemerkenswert ist der Befund, dass der Anstieg von BDNF im rechten Striatum sogar noch höher war (8,5-fach). Bekannt ist, dass kompensatorische Reaktionen auf neurodegenerative Vorgänge im Gehirn erfolgen. Ausserdem projizieren dopaminerge Neurone Fortsätze aus der Substantia nigra, dem Himareal in dem sich die Zellkörper befinden, zum Striatum der anderen Seite.

Die Konzentration eines weiteren Neurotrophins, des Glial cell line derived neurotrophic factors (GDNF) stieg nicht im linken, aber im rechten Striatum um den Faktor 12 an. Dieser Befund ist deshalb sehr interessant, weil die Infusion des Proteins GDNF in das hintere Putamen von Parkinsonpatienten ein Auswachsen von Zellfortsätzen dopaminerger Neurone und eine Verbesserung der klinischen Symptomatik bewirkte (Love et al., Nature Medicine, 2005, 11, 703).

Zusammenfassend zeigen die in vivo Experimente, dass 9-Methyl-β-Carbolin in einem etablierten Modell der Parkinson'schen Erkrankung neuroprotektiv wirkt. In diesem Modell werden überwiegend dopaminerge Nervenzellen geschädigt. Die Aussage bezieht sich deshalb nur auf diesen Nervenzelltyp. Der zugrunde liegende Mechanismus besteht in der Aktivierung von Neurotrophinen, von denen bekannt ist, dass sie eine entscheidende Rolle bei der Neubildung von Nervenzellen im Erwachsenenalter spielen.

### Beispiele 9-20: Anti-Parkinson-Test der Verbindungen A bis L

Die Verbindungen A bis L wurden so wie in Beispiel 8 beschrieben getestet. Die Testergebnisse der Verbindungen A bis L sind in folgender Tabelle im Vergleich zum 9-Methyl-β-Carbolin angegeben.

**Tabelle 1: Verbindungen A bis L im Vergleich zu 9-Methyl-β-Carbolin**

| 9-Methyl-β-Carbolin | **Standard** | |
|---|---|---|
| Verbindung A | + | |
| Verbindung B | ± | +: geringfügig besser als 9-Methyl-β-Carbolin |
| Verbindung C | ± | |
| Verbindung D | + | |
| Verbindung E | + | |
| Verbindung F | ± | ±: gleich wie 9-Methyl-β-Carbolin |
| Verbindung G | + | |
| Verbindung H | + | |
| Verbindung I | - | -: geringfügig schlechter als 9-Methyl-β-Carbolin |
| Verbindung J | ± | |
| Verbindung K | ± | |
| Verbindung L | + | |

### Beispiel 21: Synthese von 9-Methyl-β-Carbolin

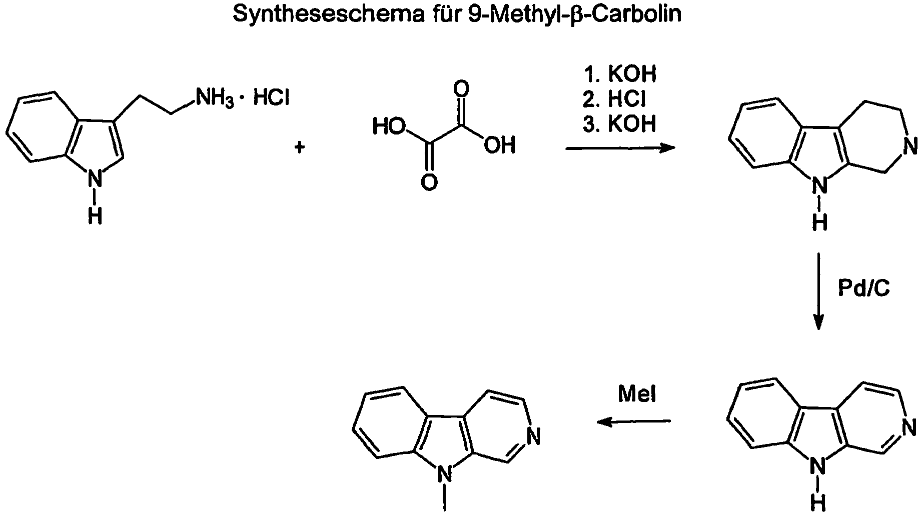

Eine gerührte Lösung von 13 g (0,0756 Mol) 1,2,3,4-Tetrahydro-β-Carbolin, hergestellt aus Tryptaminhydrochlorid und Glyoxalsäure, wie von Ho und Walker (1988) beschrieben und 2,6 g Pd/C (10%) in 600 ml Cumol wurden unter Stickstoffatmosphäre für 90 min. refluxiert. Nach Zugabe von 100 ml Ethanol wurde die heiße Lösung filtriert und die Kohle mit 3 x 30 ml heißem Ethanol extrahiert. Die vereinigten flüssigen Fraktionen wurden eingeengt und der Rückstand aus Toluol kristallisiert um 10,5 g (82%) Norharman zu erhalten. Die Methylierung in 9-Position wurde, wie in der Literatur (Ho BT, Mclsaac WM, Walker KE, Estevez V, J Pharm Sci 57: 269, 1968) beschrieben, doch mit einer verbesserten Aufarbeitung, durchgeführt: 1 g (5,95 mMol) Norharman wurden in 10 ml trockenem DMF unter Stickstoffatmosphäre gelöst. Danach wurden 0,36 g (14,9 mMol) Natriumhydrid als eine 60%ige Dispersion in Petroleum bei 0°C hinzugefügt. Nach Abkühlen der Reaktionsmischung auf Raumtemperatur wurde diese auf -10°C abgekühlt und es wurden 0,84 g (5,95 mMol) Methyliodid hinzugefügt. Nach weiterem Rühren für 12 h ließ man die Mischung erneut auf Raumtemperatur abkühlen. Alle flüchtigen Bestandteile wurden unter reduziertem Druck entfernt. Danach wurden 100 ml Wasser hinzugefügt und die Mischung wurde mit 3 x 50 ml CHCl₃ extrahiert. Die vereinigten organischen Fraktionen wurden mit 5 x 20 ml Wasser gewaschen und zur Trockne eingedampft. Der Rückstand wurde in 100 ml 2N Salzsäure aufgenommen. Um das Edukt vom gewünschten methylierten Produkt abzutrennen, wurde eine Ionenpaar-Extraktion des HCl-Salzes in CHCl₃ und in einem Flüssig/Flüssig-Extraktor für 2 Tage durchgeführt. Nach Entfernen des Lösungsmittels erhielt man 0.7 g (64%) gelbe Kristalle von 9-Methyl-β-Carboliniumhydrochlorid.

Schmpkt: 295° ; GC/MS für die freie Base: m/z = 182 (100%), 167 (5%), 140 (10%), 127 (10%), 113 (5%), 91 (10%). ¹H-NMR (HCl-Salz): δ (ppm) Methanol d4, 250 MHz: 4.06 s, 3H, N-CH₃; 7.28 - 7.35, dt, *J* = 1.2; 6.8, 1H, H6; 7.58 - 7.70, m, 2H, H7, H8; 8.13 - 8.16, d, *J* = 5.4, 1 H, H4; 8.18 - 8.21, d, *J* = 7.9, 1 H H5; 8.31 - 8.33, d, *J =* 5.4, 1H, H3; 8.89, s, 1H, H1.

### Beispiele 22-33: Herstellung der Verbindungen A bis L

Die Synthese der Verbindungen A bis L erfolgt gemäß Beispiel 21, indem die entsprechenden Alkyliodide, Alkylbromide oder Alkyltosylate eingesetzt werden. Die Ausbeuten liegen zwischen 30 und 75% der Theorie.

## Patentansprüche

1. Verwendung der Verbindungen der allgemeinen Formel (I) 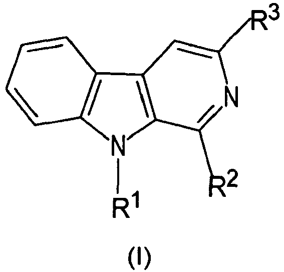 worin
**R¹** für einen der folgenden Reste steht:
-R¹⁶, -CR⁴R⁵R⁶, -X-CR⁴R⁵R⁶, -X-R⁴, -CO-O-R¹⁶;
**R²** folgende Reste bedeutet: -H, -R⁷, -CR⁹R¹⁰R¹¹, -Y-CR⁹R¹⁰R¹¹, -Y-R¹⁵, -O-R¹⁷, -S-R¹⁸, -NH-R¹⁹, -CO-NH-R²⁰, -CO-O-R²¹;
**R³** folgende Reste bedeutet: -H, -R⁸, -Z-CR¹²R¹³R¹⁴, -Z-R¹⁵, -O-R¹⁷, -S-R¹⁸, -NH-R¹⁹, -CO-NH-R²⁰, -CO-O-R²¹;
**X** ausgewählt werden kann aus -(CH₂)ₙ-, -CH=CH-, -C≡C-, -CH₂-CO-, -CO-, -CO-CH₂-;
**Y**, **Z** unabhängig voneinander bedeuten: -(CH₂)ₘ-, -CH=CH-, -C≡C-, -O-(CH₂)ₚ-, -NH-(CH₂)_{q}-, -NH-CO-, -CO-, -O-CO-;
m, p, q unabhängig voneinander eine ganze Zahl zwischen 1 und 4 darstellen;
n eine ganze Zahl zwischen 1 und 6 darstellt
**R⁴ - R¹⁴** unabhängig voneinander folgende Reste bedeuten: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅. -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇. -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R¹⁵** folgende Reste bedeutet: -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R⁴ - R⁷** und **R⁹ - R²¹** unabhängig voneinander folgende Reste bedeuten:
-CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C_{2H5}, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂. -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡-CH)₂, -CH(C≡CH)-C≡C-CH₃;
**R⁸** folgende Reste bedeutet:
-CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cycto-C₆H₁₁, cyclo-C₇H₁₃, cyclo-C₈H₁₅, -Ph, -CH₂-Ph, -CPh₃, -C₂H₅, -C₃H₇, -CH(CH₃)₂ -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)2, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃₎-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃₎-C(CH₃₎=CH_{2,} -C(CH₃)=CH-C(CH₃)=CH2, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡-C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡-C₂H₅, -C≡C-CH(CH₃)-C≡CH, -CH(CH₃)-C≡C-C≡CH, -CH(C≡CH)-CH₂-C≡CH, -C(C≡CH)₂-CH₃ -CH₂-CH(C≡CH)₂, -CH(C≡CH)-C≡C-CH₃ sowie pharmakologisch verträgliche Salze, Solvate, Hydrate, Enantiomere, Diastereomere als auch Racemate der vorgenannten Verbindungen zur Herstellung eines Medikamentes für die Behandlung und/oder Prophylaxe von Bewegungsstörungen, Bewegungsstörungen aufgrund anderer Ursachen als der Parkinson'schen Krankheit, Alzheimer, Parkinson'schen Krankheit, Hemiatrophie-Hemiparkinson, Parkinsonsyndrom aufgrund von bzw. bei Hydrocephalus (Wasserkopf), Sauerstoffmangel, Infektionen des Gehirns (Encephalitis), Vergiftungen mit Mangan, Kohlenmonoxid (CO), 1-Methyl-4-Phenyl-1,2,3,6-Tetrahydropyridin (MPTP) und Cyanid, Erkrankungen der Nebenschildrüse, Hirnverletzung, Infarkte, sowie Lewy-Körperchen Krankheit, frontotemporale Demenz, Lytico-Bodig Krankheit (Parkinsonismus-Demenz-amyotrophe Lateralsklerose), striatonigrale Degeneration, Shy-Dräger-Syndrom, sporadische olivo-ponto-cerebelläre Degeneration, progressive pallidale Atrophie, fortschreitende supranukleäre Lähmung (progressive supranuclear palsy), Huntington'sche Krankheit, x-Chromosom verknüpfte Dystonie (Morbus Lubag), mitochondriale Zytopathie mit striataler Nekrose, Neuroakanthocytose, restless legs Syndrom und Wilson'sche Krankheit.

2. Verwendung einer pharmazeutischen Zusammensetzung umfassend mindestens eine Verbindung gemäß Anspruch 1 und/oder pharmakologisch verträgliche Salze davon und einen pharmakologisch verträglichen Träger, Hilfsstoff und/oder Lösungsmittel zur Herstellung eines Medikamentes für die Behandlung und/oder Prophylaxe von Bewegungsstörungen, Bewegungsstörungen aufgrund anderer Ursachen als der Parkinson'schen Krankheit, Alzheimer, Parkinson'schen Krankheit, Hemiatrophie-Hemiparkinson, Parkinsonsyndrom aufgrund von bzw. bei Hydrocephalus (Wasserkopf), Sauerstoffmangel, Infektionen des Gehirns (Encephalitis), Vergiftungen mit Mangan, Kohlenmonoxid (CO), 1-Methyl-4-Phenyl-1,2,3,6-Tetrahydropyridin (MPTP) und Cyanid, Erkrankungen der Nebenschildrüse, Hirnverletzung, Infarkte, sowie Lewy-Körperchen Krankheit, frontotemporale Demenz, Lytico-Bodig Krankheit (Parkinsonismus-Demenz-amyotrophe Lateralsklerose), striatonigrale Degeneration, Shy-Dräger-Syndrom, sporadische olivo-ponto-cerebelläre Degeneration, progressive pallidale Atrophie, fortschreitende supranukleäre Lähmung (progressive supranuclear palsy), Huntington'sche Krankheit, x-Chromosom verknüpfte Dystonie (Morbus Lubag), mitochondriale Zytopathie mit striataler Nekrose, Neuroakanthocytose, restless legs Syndrom und Wilson'sche Krankheit.

3. Verwendung der pharmazeutischen Zusammensetzung gemäß Anspruch 2 in Form von transdermalen Applikationssystemen, Pflastern, Filmen, Tropfen, Mundspray, Nasenspray, Pillen, Tabletten, Filmtabletten, Schichttabletten, Zäpfchen, Gelen, Salben, Sirup, Inhalationspulvern, Granulaten, Suppositorien, Emulsionen, Dispersionen, Mikrokapseln, Kapseln, Puder oder Injektionslösungen.

4. Verwendung der pharmazeutischen Zusammensetzung gemäß Anspruch 2 oder 3 geeignet zur Inhalation oder zur intravenösen, intraperitonealen, intramuskulären, subkutanen, mucokutanen, oralen, rektalen, transdermalen, topikalen, bukkal, intradermalen, intragastralen, intrakutanen, intranasalen, intrabuccalen, perkutanen oder sublingualen Applikation.

5. Verwendung der Verbindungen gemäß Anspruch 1 als Additivum zur Beschleunigung des Wachstums von Zellen in Zellkulturmedien, in Nährmedien oder in Fermentationsmedien.

6. Verwendung der Verbindungen gemäß Anspruch 5, wobei es sich bei den Zellen um humane und tierische Zellkulturen von Knochenzellen, Knorpelzellen, Fibroblasten, neuronalen Zellen, Gliazellen, dopaminergen Zellen, Fettzellen, Muskelzellen, Herzmuskelzellen, Hautzellen, Hepatozyten, Mesenchymzellen, gonadotropen Zellen, Cumuluszellen, Blutzellen, Epithelzellen, Endothelzellen, Basalzellen, adulten und embryonalen Stammzellen und multipotenten Vorläuferzellen handelt.

7. Zellkulturmedien, Nährmedien oder Fermentationsmedien enthaltend als Additivum mindestens eine Verbindung der allgemeinen Formel (I) 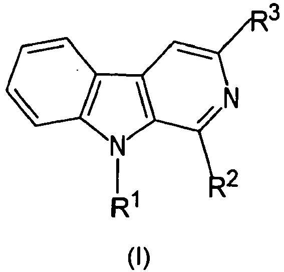 worin
**R¹** für einen der folgenden Reste steht:
-R¹⁶, -CR⁴R⁵R⁶, -X-CR⁴R⁵R⁶, -X-R⁴, -CO-O-R¹⁶;
**R²** folgende Reste bedeutet: -H, -R⁷, -CR⁹R¹⁰R¹¹, -Y-CR⁹R¹⁰R¹¹, -Y-R¹⁵, -O-R¹⁷, -S-R¹⁸, -NH-R¹⁹, -CO-NH-R²⁰, -CO-O-R²¹;
**R³** folgende Reste bedeutet: -Z-CR¹²R¹³R¹⁴, -O-R¹⁷, -S-R¹⁸, -NH-R¹⁹, -CO-NH-R²⁰;
**X** ausgewählt werden kann aus -(CH2)ₙ-, -CH=CH-, -C≡C-, -CH₂-CO-, -CO-, -CO-CH₂-;
**Y, Z** unabhängig voneinander bedeuten: -(CH₂)ₘ-, -CH=CH-, -C≡C-, -O-(CH₂)ₚ-, -NH-(CH₂)_{q}-, -NH-CO-, -CO-, -O-CO-;
m, p, q unabhängig voneinander eine ganze Zahl zwischen 1 und 4 darstellen;
n eine ganze Zahl zwischen 1 und 6 darstellt
**R⁴** - **R¹⁴** unabhängig voneinander folgende Reste bedeuten: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R¹⁵** folgende Reste bedeutet: -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R⁴** - **R⁷** und **R⁹ - R²⁰** unabhängig voneinander folgende Reste bedeuten: -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C=CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
**R⁸** folgende Reste bedeutet:
-CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅. -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂. -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C=C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃; sowie pharmakologisch verträgliche Salze, Solvate, Hydrate, Enantiomere, Diastereomere als auch Racemate der vorgenannten Verbindungen.

## Claims

1. Use of compounds of the general formula (I) 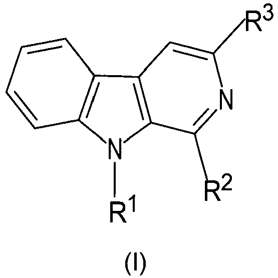 wherein
**R¹** means one of the following residues:
-R¹⁶, -CR⁴R⁵R⁶, -X-CR⁴R⁵R⁶, -X-R⁴, -CO-O-R¹⁶;
**R²** represents the following residues: -H, -R⁷, -CR⁹R¹⁰R¹¹, -Y-CR⁹R¹⁰R¹¹, -Y-R¹⁵, -O-R¹⁷, -S-R¹⁸, -NH-R¹⁹, -CO-NH-R²⁰, -CO-O-R²¹;
**R³** represents the following residues: -H, -R⁸, -Z-CR¹²R¹³R¹⁴, -Z-R¹⁵, -O-R¹⁷, -S-R¹⁸, -NH-R¹⁹, -CO-NH-R²⁰, -CO-O-R²¹;
**X** can be selected from, -(CH₂)ₙ-, -CH=CH-, -C≡C-, -CH₂-CO-, -CO-, -CO-CH₂-;
**Y, Z** represent independently of each other: -(CH₂)ₘ-, -CH=CH-, -C≡C-, -O-(CH₂)ₚ-, -NH-(CH₂)_{q}-, -NH-CO-, -CO-, -O-CO-;
m, p, q represent independently of each other an integer between 1 and 4;
n represents an integer between 1 and 6
**R⁴** - **R¹⁴** represent independently of each other the following residues: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R¹⁵** represents the following residues: -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R⁴ - R⁷** and **R⁹ - R²¹** represent independently of each other the following residues:
-CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂₋C(CH₃)₃, -CH(C₂H₅)₂, -C₂H₄₋CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH2-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
**R⁸** represents the following residues:
-CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₂H₅, -C₃H₇, -CH(CH₃)₂, **-**C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, **-**C₃H₆**-**CH(CH₃)₂**,** -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
and pharmaceutically acceptable salts, solvates, hydrates, enantiomers, diastereomers as well as racemats of the afore-mentioned compounds for the preparation of a medicament for the treatment and/or prophylaxis of movement disorders, movement disorders due to other causes than Parkinson's disease, Alzheimer's disease, Parkinson's disease, hemiparkinsonism hemiatrophy, Parkinson syndrom due to or during hydrocephalus (water in the brain), oxygen deficiency, cerebral infections (encephalitis), manganese intoxication, carbon monoxide (CO) intoxication, 1-methyl-4-phenyl-1,2,3,6-tetrahydropyridine (MPTP) intoxication and cyanide intoxication, parathyroid diseases, brain injury, infarcts, as well as Lewy bodies disease, frontotemporal dementia (Pick's disease), Lytico-Bodig disease (Parkinsonism-dementia-amyotrophic lateral sclerosis), striatonigral degeneration, idiopathic orthostatic hypotension (Shy-Drager syndrom), sporadical olivopontocerebellar degeneration, progressive atrophy of globus pallidus, progressive supranuclear palsy, Huntington's disease, X chromosome-linked dystonia (Morbus Lubag), mitochondrial cytopathy with striatal necrosis, neuroacanthocytosis, restless legs syndrome and Wilson's disease.

2. Use of a pharmaceutical composition comprising at least one compound according to claim 1 and/or pharmaceutically acceptable salts thereof and a pharmaceutical acceptable carrier, excipient, and/or solvent for the preparation of a medicament for the treatment and/or the prophylaxis of movement disorders, movement disorders due to other causes than Parkinson's disease, Alzheimer's disease, Parkinson's disease, hemiparkinsonism hemiatrophy, Parkinson syndrom due to or during hydrocephalus (water in the brain), oxygen deficiency, cerebral infections (encephalitis), manganese intoxication, carbon monoxide (CO) intoxication, 1-methyl-4-phenyl-1,2,3,6-tetrahydropyridine (MPTP) intoxication and cyanide intoxication, parathyroid diseases, brain injury, infarcts, as well as Lewy bodies disease, frontotemporal dementia (Pick's disease), Lytico-Bodig disease (Parkinsonism-dementia-amyotrophic lateral sclerosis), striatonigral degeneration, idiopathic orthostatic hypotension (Shy-Drager syndrom), sporadical olivopontocerebellar degeneration, progressive atrophy of globus pallidus, progressive supranuclear palsy, Huntington's disease, X chromosome-linked dystonia (Morbus Lubag), mitochondrial cytopathy with striatal necrosis, neuroacanthocytosis, restless legs syndrome and Wilson's disease.

3. Use of the pharmaceutical composition according to claim 2 in the form of transdermal application systems, band aids, films, drops, oral spray, nasal spray, pills, tablets, coated tablets, layered tablets, suppositories, gels, iontments, syrup, inhalation powder, pellets, emulsions, dispersions, micro capsules, capsules, powder or injection solutions.

4. Use of the pharmaceutical composition according to claim 2 or 3 suitable for inhalation or intravenous, intraperitoneal, intramuscular, subcutaneous, mucocutaneous, oral, rectal, transdermal, local, buccal, intradermal, intragastric, intracutaneous, intranasal, intrabuccal, percutaneous, or sublingual application.

5. Use of the compounds according to claim 1 as an additive for acceleration of cell growth in cell culture medium, in growth medium or in fermentation medium.

6. Use of compounds according to claim 5, wherein the cells are human and animal cell cultures of bone cells, cartilage cells, fibroblasts, neuronal cells, glial cells, dopaminergic cells, fat cells, myocytes, cardiomyocytes, skin cells, hepatocytes, mesenchymal cells, gonadotrope cells, cumulus cells, blood cells, epithelial cells, endothelial cells, basal cells, adult and embryonic stem cells and multipotent progenitor cells.

7. Cell culture media, growth media or fermentation media containing as an additive at least one compound of the general formula (I) 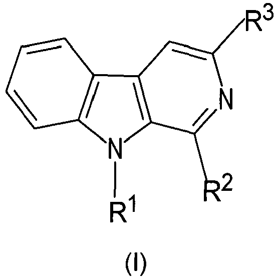 wherein
**R¹** means one of the following residues:
-R¹⁶, -CR⁴R⁵R⁶, -X-CR⁴R⁵R⁶, -X-R⁴, -CO-O-R¹⁶;
**R²** represents the following residues: -H, -R⁷, -CR⁹R¹⁰R¹¹, -Y-CR⁹R¹⁰R¹¹, -Y-R¹⁵, -O-R¹⁷, -S-R¹⁸, -NH-R¹⁹, -CO-NH-R²⁰, -CO-O-R²¹;
**R³** represents the following residues: -Z-CR¹²R¹³R¹⁴, -O-R¹⁷, -S-R¹⁸, -NH-R¹⁹, -CO-NH-R²⁰;
**X** can be selected from, -(CH₂)ₙ-, -CH=CH-, -C≡C-, -CH₂-CO-, -CO-, -CO-CH₂-;
**Y**, **Z** represent independently of each other: -(CH₂)ₘ-, -CH=CH-, -C≡C-, -O-(CH₂)ₚ-, -NH-(CH₂)_{q}-, -NH-CO-, -CO-, -O-CO-;
m, p, q represent independently of each other an integer between 1 and 4;
n represents an integer between 1 and 6
**R⁴** - **R¹⁴** represent independently of each other the following residues: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R¹⁵** represents the following residues: -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH(CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R⁴** - **R⁷** and **R⁹** - **R²¹** represent independently of each other the following residues:
-CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃; cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅**,** -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
**R⁸** represents the following residues:
-CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
and pharmaceutically acceptable salts, solvates, hydrates, enantiomers, diastereomers as well as racemats of the afore-mentioned compounds.

## Revendications

1. Utilisation des composés de formule générale (I) 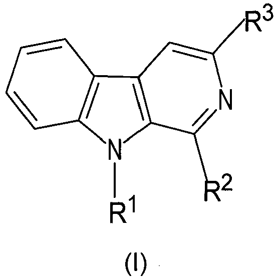 où
**R¹** représente l'un des restes:
-R¹⁶, -CR⁴R⁵R⁶, -X-CR⁴R⁵R⁶, -X-R⁴, -CO-O-R¹⁶;
**R²** indique l'un des restes suivant : -H, -R⁷,-CR⁹R¹⁰R¹¹, -Y-CR⁹R¹⁰R¹¹, -Y-R¹⁵ , -O-R¹⁷, -S-R¹⁸, -NH-R¹⁹, -CO-NH-R²⁰, -CO-O-R²¹;
**R³** indique l'un des restes suivant : -H, -R⁸, -Z-CR¹²R¹³R¹⁴, -Z-R¹⁵, -O-R¹⁷, -S-R¹⁸, -NH-R¹⁹, -CO-NH-R²⁰,-CO-O-R²¹;
**X** peut être sélectionné parmi -(CH₂)ₙ-, -CH=CH-, -C≡C-, -CH₂-CO-, -CO-, -CO-CH₂-;
**Y, Z** indiquent indépendamment: -(CH₂)ₘ-, -CH=CH-, -C≡C-, -O-(CH₂)ₚ-, -NH-(CH₂)_{q}-, -NH-CO-, -CO-, -O-CO-;
m, p et q représentent indépendamment un entier de 1 à 4 ;
n, représente un entier de 1 à 6
**R⁴ - R¹⁴** représentent indépendamment l'un des restes suivant: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇ -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H_{5,} -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R¹⁵** représente l'un des restes suivant: -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H,₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R⁴ - R⁷** et **R⁹ - R²¹** représentent indépendamment l'un des restes suivant: -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
**R⁸** représente l'un des restes suivant:
-CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂; -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
ainsi que sels pharmacologiquement acceptables, solvates hydrates, énantiomères, diastéréomères et racémates des composés susmentionnés pour la fabrication d'une composition pharmaceutique pour le traitement ou la prophylaxie des troubles du mouvement, troubles du mouvement des raisons autres que la maladie de Parkinson, maladie d'Alzheimer, maladie de Parkinson, hémiatrophie-hémiparkinson, de syndromes parkinsoniens à cause de l'hydrocéphalie, manque d'oxygène, infections cérébrales (encéphalite), intoxication par le manganèse, le monoxyde de carbone (CO), 1 - méthyle 4 - phényl 1,2,3,6-tétrahydro pyridine (MPTP) et cyanure, maladie parathyroïdienne, lésion cérébrale, infarctus, et maladie avec les corps de Lewy, démence associée à la détérioration des lobes frontal et temporal, syndrome de Guam (démence parkinsonnienne-sclérose latérale amyotrophique), la dégénérescence striatonigrique (DSN), le syndrome de Shy-Drager (SSD), sporadique dégénérescence olivo-ponto-cérébelleuse, paralysie supranucléaire progressif, atrophie progressif du globus pallidus, chorée de Huntington, dystonie associé au chromosome X (Morbus Lubag), cytopathie mitochondriale avec nécrose strié, neuroacanthocytose, syndrome des jambes sans repos et la maladie de Wilson.

2. Utilisation d'une composition pharmaceutique comprenant au moins un composé selon la revendication 1 ou un sel pharmaceutiquement acceptable de celui et un additif neutres d'un point de vue pharmaceutique, des excipients, et/ou solvant pour la fabrication d'une composition pharmaceutique pour le traitement ou la prophylaxie des troubles du mouvement, troubles du mouvement des raisons autres que la maladie de Parkinson, maladie de Parkinson, maladie d'Alzheimer, maladie de Parkinson, hémiatrophie-hémiparkinson, de syndromes parkinsoniens à cause de l'hydrocéphalie, manque d'oxygène, infections cérébrales (encéphalite), Intoxication par le manganèse, le monoxyde de carbone (CO), 1 - méthyle 4 - phényl 1,2,3,6-tétrahydro pyridine (MPTP) et cyanure, maladie parathyroïdienne, lésion cérébrale, infarctus, et maladie avec les corps de Lewy, démence associée à la détérioration des lobes frontal et temporal, syndrome de Guam (démence parkinsonnienne-sclérose latérale amyotrophique), la dégénérescence striatonigrique (DSN), le syndrome de Shy-Drager (SSD), sporadique dégénérescence olivo-ponto-cérébelleuse, paralysie supranucléaire progressif, atrophie progressif du globus pallidus, chorée de Huntington, dystonie associé au chromosome X (Morbus Lubag), cytopathie mitochondriale avec nécrose strié, neuroacanthocytose, syndrome des jambes sans repos et la maladie de Wilson.

3. Utilisation d'une composition pharmaceutique selon la revendication 2 sous form des systèmes d'administration transdermiques, emplâtres, films, gouttes, spray buccal, spray nasal, pilules, comprimés, comprimés pelliculés, comprimés à couches, luette, gels, crèmes, sirop, poudre à inhaler, granulats, suppositoires, émulsions, dispersions, micro capsules, capsules, poudre ou solutions de l'injection.

4. Utilisation d'une composition pharmaceutique selon la revendication 2 ou 3 appropriée pour administration par d'inhalation, ou administration intraveineuse, intrapéritonéal, intramusculaire, sous-cutané, mucocutanées, orale, rectale, transdermique, local, buccal, intradermique, intragastrique, intracutanées, intranasal, intrabuccal, percutanées ou sublinguale.

5. Utilisation des composés selon la revendication 1 comme d'additive pour accélération de la croissance cellulaire au milieu de culture cellulaire, au milieu de culture ou fermentation.

6. Utilisation des composés selon la revendication 5, qui les cellules sont des cultures de cellulaire humaines et animales des cellules osseuses, des cellules cartilages, des fibroblastes, des cellules neuronales, des cellules gliales, des cellules dopaminergique, des cellules adipeuses, des cellules musculaires, des cellules musculaires cardiaques, des cellules de la peau, des hépatocytes, des cellules de mésenchyme, des cellules gonadotrope, des cumulus, des cellules sanguines, des cellules épithéliales, des cellules basiques, des cellules souches adultes et embryonnaires et des cellules multipotentes.

7. Milieu de culture cellulaire, milieu de culture ou de fermentation qui comprend en tant qu'additif au moins un composé de formule générale (I) 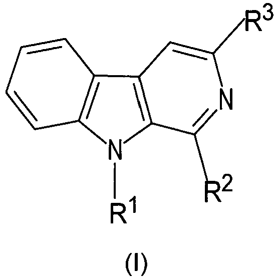 où
**R¹** représente l'un des restes:
-R¹⁶, -CR⁴R⁵R⁶, -X-CR⁴R⁵R⁶, -X-R⁴, -CO-O-R¹⁶;
**R²** indique l'un des restes suivant: -H, -R⁷,-CR⁹R¹⁰R¹¹, -Y-CR⁹R¹⁰R¹¹, -Y-R¹⁵, -O-R¹⁷, -S-R¹⁸, -NH-R¹⁹, -CO-NH-R²⁰, -CO-O-R²¹;
**R³** indique l'un des restes suivant: -Z-CR¹²R¹³R¹⁴, -O-R¹⁷, -S-R¹⁸, -NH-R¹⁹, -CO-NH-R²⁰,
**X** peut être sélectionné parmi -(CH₂)ₙ-, -CH=CH-, -C≡C-, -CH₂-CO-, -CO-, -CO-CH₂-;
**Y, Z** indiquent indépendamment: -(CH₂)ₘ-, -CH=CH-, -C≡C-, -O-(CH₂)ₚ-, -NH-(CH₂)_{q}-, -NH-CO-, -CO-, -O-CO-;
m, p et q représentent indépendamment un entier de 1 à 4 ;
n, représente un entier de 1 à 6
**R⁴ - R¹⁴** représentent indépendamment l'un des restes suivant: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -CI, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R¹⁵** représente l'un des restes suivant: -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)_{2]}, -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃,
**R⁴** - **R**⁷ et **R⁹** - **R²¹** représentent indépendamment l'un des restes suivant: -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH2, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
**R⁸** représente l'un des restes suivant:
-CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH2, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
ainsi que sels pharmacologiquement acceptables, solvates, hydrates, énantiomères, diastéréomères, ainsi que des racémates des composés susmentionnés.
